# EUROPEAN PATENT SPECIFICATION

(11) **EP 2 928 513 B1**
(45) Date of publication and mention of the grant of the patent: **06.06.2018**
(21) Application number: 13861358.3
(22) Date of filing: 06.12.2013
(51) Int. Cl.: A61L 27/52, A61L 27/22, C07K 14/76

(54) **PEPTIDE-ALBUMIN HYDROGEL PROPERTIES AND ITS APPLICATIONS**
PEPTID-ALBUMIN-HYDROGEL-EIGENSCHAFTEN UND DEREN ANWENDUNGEN
PROPRIÉTÉS D'HYDROGEL PEPTIDE-ALBUMINE ET SES APPLICATIONS

(30) Priority: 07.12.2012 US 201261734708 P
(43) Date of publication of application: 14.10.2015
(73) Proprietor: Kansas State University Research Foundation, Manhattan, KS 66502 (US)
(72) Inventor: SUN, Xiuzhi S., Manhattan, Kansas 66503 (US); HUANG, Hongzhou, Manhattan, Kansas 66502 (US); NGUYEN, Thu Annelise, Manhattan, Kansas 66503 (US)
(74) Representative: Uexküll & Stolberg
(86) International application number: PCT/US2013/073645
(87) International publication number: WO 2014/089472

(56) References cited:
- WO-A1-2009/012449
- WO-A2-02/062969
- WO-A2-2008/039483
- WO-A2-2011/002249
- WO-A2-2011/112856
- WO-A2-2012/074588
- US-A1- 2011 002 880
- AURORE SCHNEIDER ET AL: "Self-Assembling Peptide Nanofiber Scaffolds Accelerate Wound Healing", PLOS ONE, vol. 3, no. 1, 9 January 2008 (2008-01-09) , page e1410, XP055282482, DOI: 10.1371/journal.pone.0001410

## Description

### SEQUENCE LISTING

The following application contains a sequence listing in computer readable format (CRF), submitted as a text file in ASCII format entitled "SequenceListing," created on December 4, 2013, as 17.6 KB. The content of the CRF is hereby incorporated by reference.

### BACKGROUND OF THE INVENTION

### Field of the Invention

The present invention relates to self-assembling peptide-albumin hydrogels.

### Description of Related Art

Using peptide hydrogels as injectable materials for tissue engineering and other biotechnological applications has been an important discovery made over the past few decades. Because of its high water content and polymer network, peptide hydrogels are a promising material for storage and transfer of proteins without significant loss of their biological activity. A sol-gel transformation occurs when peptide molecules self-assemble into a well-defined nanofiber network that traps water molecules. Because this transformation occurs at specific temperatures and pHs, peptide hydrogel precursors may be injected into the body in the liquid phase and converted into hydrogels when physiological conditions (e.g., pH, temperature) change *in vivo*, which is important for injectable applications.

There is particularly a need for injectable hydrogels that are shear thinning and have rapid recovery characteristics. Such a hydrogel should have a storage modulus that decreases sharply under a shearing force, but that recover after the force is removed. Improved methods of forming peptide hydrogels are also needed, which avoid the need for chemical or environmental modification of the hydrogel solution to induce hydrogel formation.

US2011/002880 A1 discloses bioabsorbable materials for wound healing comprising a self-assembling peptide hydrogel. The peptide is an amphiphilic peptide. The material further comprises e.g. a blood component, such as albumin. The material self-assembles to form a 3-dimensional nanofiber matrix.

### SUMMARY OF THE INVENTION

The present invention is broadly concerned with peptide-albumin hydrogels having a self-assembling, 3-dimensional nanofiber matrix. The nanofiber matrix comprises (consists essentially or even consists of) an amphiphilic peptide and albumin. The amphiphilic peptide comprises a terminal hydrophobic region, a central turning region, and a terminal hydrophilic region.

Methods of forming a peptide-albumin hydrogel are also described herein. The methods comprise providing a peptide solution comprising a peptide dispersed, dissolved, or suspended in a solvent system, and mixing a source of albumin with the peptide solution at room temperature to form a peptide-albumin solution. The peptide is amphiphilic and comprises a terminal hydrophobic region, a central turning region, and a terminal hydrophilic region. The peptide and albumin self-assemble into the peptide-albumin hydrogel without adjusting the pH, temperature, salt, or ion composition of the peptide-albumin solution. The peptide-albumin hydrogels are disclosed herein for use in methods of delivering an active agent to a patient. The methods comprise administering to the patient a peptide-albumin hydrogel according to any of the embodiments described herein, wherein the active agent is encapsulated in the hydrogel matrix.

### BRIEF DESCRIPTION OF THE DRAWINGS

Figure (Fig.) 1 shows TEM images of peptide albumin mixture: a. h9e/BSA mixture, b. h9e/HSA mixture;
Fig. 2 shows graphs illustrating the mechanical properties of h9e/BSA hydrogel;
Fig. 3 shows graphs illustrating the mechanical properties of h9e/HSA hydrogel;
Fig. 4 illustrates peptide hydrogelation in MEM. A. Proposed mechanism of MEM-induced h9e peptide self-assembling hydrogelation (SEM image showing the nanofiber scaffold of the hydrogel matrix). B. & C. Storage modulus G' of 1, 2, and 3 mM peptide hydrogel during the hydrogelation at 37 °C. D. SEM image of 1 mM peptide hydrogel. E. SEM image of 3 mM peptide hydrogel;
Fig. 5 illustrates the dynamic rheological study of h9e hydrogel. A. Storage modulus G' of shear-thinning and recovery test of 1, 2, and 3 mM peptide hydrogel. B. Four times amplitude sweep test with shear strain from 1% to 500% and 1- 5-, and 10-min breaks. C. Multiple times delivery of peptide hydrogel via pipette; hydrogel was shear thinning but reassembled quickly without permanently destroying hydrogel architecture. D. Temperature profile test of 1, 2, and 3 mM peptide hydrogel between 4 °C and 50 °C;
Fig. 6 illustrates the dynamic rheological study of h9e-MSC hydrogel. A. G' of 1 mM, 2 mM and 3mM h9e-MSC hydrogel during hydrogel formation. B. G' and G" of shear-thinning and recovery test of 2 mM h9e-MSC hydrogel. C. G' and G" of 2 mM h9e-MSC hydrogel after 20 folds dilution;
Fig. 7 illustrates the dynamic rheological study of h9e-2i hydrogel. A. G' of 1 mM, 2 mM and 3mM h9e-2i hydrogel during hydrogel formation. B. G' and G" of shear-thinning and recovery test of 2 mM h9e-2i hydrogel. C. G' and G" of 2 mM h9e-2i hydrogel after 20 folds dilution; and
Fig. 8 illustrates the stability of h9e-2i hydrogle and h9e solution. a. G' of h9e-2i hydrogel from Day 1 to Day 7. b. The hydrogel formation of h9e-2i mixture after the h9e solution was stored at 4 °C refrigerator for 1, 6, 18 and 24 days.

### DETAILED DESCRIPTION

The present invention is concerned with self-assembled hydrogels and methods of making and using the same. The hydrogel matrix network comprises a peptide and albumin which make up the 3-dimensional nanofibrous network of the hydrogel structure. The peptide-albumin hydrogels are characterized by a "reversible" hydrogel matrix, which means that the 3-dimensional nanofibrous matrix is shear thinning (i.e., the viscosity decreases with an increase in the rate of shear stress applied to the gel), but recovers quickly after gel destruction, as discussed in more detail below. The hydrogels are useful in various applications, including as scaffolds for tissue engineering, 3-dimensional (3-D) cell cultures, drug delivery and encapsulation of therapeutic agents (cells, molecules, drugs, compounds), injectables (including those that gel *in situ*, such as hemostatic compositions), wound dressings, pharmaceutical carriers or vehicles, cell transplantation, cell storage, virus culture, virus storage, and the like.

The peptide-albumin hydrogels have a uniform internetwork morphology with a porous structure and open cells. The average cell size of the hydrogel matrix will be from about 10 µm to about 80 µm, preferably from about 20 µm to about 60 µm, and more preferably from about 30 µm to about 50 µm, as observed under a scanning electron microscope. The average pore size will range from about 50 to about 200 nm. The hydrogel peptides are in the form of peptide nanofibers having an average diameter of from about 3 nm to about 30 nm, preferably from about 5 nm to about 20 nm, and more preferably from about 8 nm to about 15 nm, as measured under a transmission electron microscope. The peptide nanofibers have an average length of from about 0.3 µm to about 5 µm, preferably from about 0.8 µm to about 3 µm, and more preferably from about 1 µm to about 2 µm.

The peptide-albumin hydrogels have a storage modulus (associated with gel strength) of at least 50 Pa, preferably at least about 100 Pa, and more preferably from about 100 Pa to 10,000 Pa. It will be appreciated that by varying the peptide and albumin concentrations, the strength of the particular hydrogel can be tuned to the desired application for the gel. For example, for an injectable hydrogel, the hydrogel matrix will have a storage modulus of from about 50 Pa to about 3,000 Pa and preferably from about 70 Pa to about 1,000 Pa. In some embodiments, such as scaffolding, very strong hydrogels can be formed, having a storage modulus of at least about 300 Pa, preferably from about 500 Pa to about 10,000 Pa, and even more preferably from about 1,000 Pa to about 5,000 Pa. These gel strengths are based upon a neutral pH (about 7) and a temperature of about room temperature (aka "ambient temperature" or about 20-25°C).

As noted above, the hydrogel matrix is reversible. This means that after gel destruction by subjecting the gel to a sufficient mechanical force (e.g., shear thinning), the hydrogels have a % recovery of at least 60%, preferably at least about 80%, more preferably at least about 90%, and even more preferably about 100% in less than 10 minutes, preferably in less than about 5 minutes, and more preferably in less than about 2 minutes (after removing the shear stress from the destroyed gel). As used herein, the "% recovery" of the hydrogel is the percentage of the original storage modulus (i.e., before gel destruction) achieved by the gel after destruction and re-hydrogelation. In other words, shear thinning only temporarily destroys the gel structure or architecture. Shear thinning can be carried out using various mechanical forces that impose a shear strain or shear stress on the hydrogel, such as pipetting, centrifugation, vibration, injection, spraying, filtration, and the like. As noted above, reassembly of the gel or hydrogelation reoccurs quickly after shear thinning and destruction of the gel structure (i.e., after removal of or stopping the application of mechanical force to the destroyed gel). This recovery property also persists even after destroying the gel structure multiple times. Advantageously, the destroyed matrix after shear thinning can also be diluted with solvent to a substantially liquid solution (i.e., G' < 0.2 Pa) to stop the recovery process. In other words, when diluted to a peptide concentration of less than about 0.1% by weight, the peptide-albumin solution remains in a low-viscosity liquefied form and does not rapidly reassemble. This technique is useful for isolating cells or other components cultured in the hydrogel, such as by separating (e.g., by centrifugation) the liquefied peptide-albumin solution from the cells. This process can be enhanced by maintaining the liquefied and diluted peptide-albumin solution at a low temperature (about 4 °C) during separation.

The inventive gels are water soluble and temperature stable up to about 90°C. As used herein, "water soluble" means the gels can be diluted with water after formation, and "temperature stable" means that the hydrogel is not denatured at temperatures ranging from about 1 °C to about 90 °C. Advantageously, unlike other types of gels, the storage modulus of the inventive peptide-albumin hydrogels increases as temperature is increased.

The hydrogels are prepared by combining the peptides with a source of albumin. As used herein, a "source of albumin" refers to one or more types of (purified) albumin that can be directly combined with the peptides, a composition containing one or more types of albumin, as well as albumin derivatives.

In one or more embodiments, the method involves forming or providing a solution of peptides according to the invention. The peptide solution comprises (consists essentially or even consists of) the peptides suspended, dispersed, or dissolved in a solvent system at levels of at least about 0.1%, preferably from about 0.1% to about 5% by weight, more preferably from about 0.3% to about 3.5% by weight, and even more preferably from about 0.5% to about 2% by weight, based upon the total weight of the solution taken as 100% by weight. Dried (e.g., freeze-dried) peptides are suitable for used in the invention and can be mixed with the solvent system to create the peptide solution. The peptide solution has a pH of from about 6 to about 8, preferably from about 6.5 to about 7.5, more preferably from about 7 to about 7.5, and even more preferably about 7. Suitable solvent systems include aqueous alkaline solutions, such as sodium bicarbonate, sodium hydroxide, potassium hydroxide, and mixtures thereof in water.

The peptide solution is combined with a source of albumin, such as a composition comprising (consisting essentially of or even consisting of) albumin. Suitable types of albumin for use in the invention include albumin isolated, extracted, and/or purified from plant or animal sources, as well as synthesized albumin, such as recombinant/transgenic albumin (e.g., human albumin expressed in a plant system), and derivatives thereof (e.g., modified albumins, such as biotin-labeled, acetylated, glycated, nitrated, etc.). The albumin itself can be directly added to the peptide solutions, or it can be provided as part of a composition that contains albumin. Examples of such compositions include serum, serum-supplemented cell media (e.g., Minnimum Essential Medium (MEM), Dulbecco's modified Eagle's medium (DMEM), Roswell Park Memorial Institute medium (RPMI), and Leibovitz medium), CMRL 1066 (Sigma), and the like. In one or more embodiments, the source of albumin can be mixed with a solvent system, such as water, to form a solution comprising albumin (or a source of albumin). This solution is then mixed with the peptide solution, preferably at about room temperature. The resulting peptide-albumin solution has a substantially neutral pH of from 6 to 8, preferably from about 6.5 to about 7.5, more preferably from about 7 to about 7.5, and even more preferably about 7.

The level of albumin used is at least about 0.1% by weight, preferably from about 0.5% by weight to about 20% by weight, and more preferably from about 1% by weight to about 10% by weight, based upon the total weight of the peptide-albumin solution taken as 100% by weight. The level of peptide used will vary depending upon the desired function of the hydrogel. In one or more embodiments, the peptide concentration is from about 0.1% by weight to about 10% by weight, more preferably from about 0.15% by weight to about 5% by weight, and even more preferably from about 0.2% by weight to about 1% by weight, based upon the total weight of the peptide-albumin solution taken as 100% by weight. However, for wound healing or hemostatic applications, higher peptide concentrations above 1% by weight may be more preferred (e.g., from about 3 to about 5% by weight) to facilitate blood clotting and stop bleeding. The gel typically comprises from about 0.1% to about 3% by weight of the peptide, preferably from about 0.25% to about 1.5% by weight of the peptide, and more preferably from about 0.5% to about 1% by weight of the peptide, based on the total weight of the gel taken as 100% by weight. In either the solution or gel, the weight ratio of peptide to albumin is preferably from 100:1 to 1:100, more preferably from about 10:1 to about 1:10, and even more preferably from about 2:1 to about 1:2.

It will be appreciated that the desired properties of the hydrogel can be varied by modifying the relative concentration of peptide and albumin in the peptide-albumin solution. For example, higher albumin concentrations can be used to induce very rapid gel formation, while higher peptide concentrations can be used to form higher strength gels. Regardless, the hydrogel is considered formed once G' (storage modulus) is greater than G" (storage loss). In other words, the composition is considered a hydrogel when it reaches self-supporting strength and is not susceptible to deformation merely due to its own internal forces. The hydrogel typically forms in less than 120 minutes after combining the peptide and the albumin, preferably less than about 60 minutes, and more preferably from about 15 to about 30 minutes. Again, it will be understood that these parameters can be varied by modifying the albumin and peptide concentrations.

Advantageously, hydrogelation is induced upon mixing the peptide solution with the albumin solution, without any further modifications to the system. That is, the preparation method does not require and is preferably essentially free of modifications or adjustments to the pH of the system (e.g., by adding a buffer), the chemical composition (e.g., by adding ions), or the temperature of the system. In other words, unlike other hydrogelation techniques, the inventive method is preferably essentially free of chemical or environmental modifications to the peptide-albumin solution for hydrogel formation. As used here, "essentially free" means that the omitted ingredients or steps are not intentionally added or carried out to achieve hydrogelation, although it is appreciated that incidental impurities or ancillary steps may be included that do not otherwise modify the hydrogel and are encompassed by the invention. The resulting hydrogels are also preferably essentially free of one or more of synthetic polymers, polysaccharides, lipids, undesirable buffers (e.g., citrate/lactate buffer, etc.), and the like.

As noted above, the hydrogels have various uses, including as scaffolding for tissue engineering, drug delivery, and the like. Advantageously, active agents, including therapeutics, such as small molecule drugs and/or biologics (e.g., enzymes and other proteins and peptides, and DNA and RNA fragments), can be encapsulated in the hydrogel simply by adding the active agent to the peptide-albumin solution, such that encapsulation takes place *in situ* during hydrogelation. Due to the neutral pH (physiological pH) of the peptide-albumin solution and the fact that chemical and/or environmental modifications are not carried out on the system to induce hydrogelation, this encapsulation technique is particularly well-suited to encapsulating biologic therapeutic agents requiring physiological conditions. The hydrogel can be used in pharmaceutically acceptable compositions as a delivery vehicle for administration of the active agent to a subject (e.g., orally, intravenously, subcutaneously, intramuscularly, nasally, etc.).

The hydrogels can also be used as hemostatic compositions to stop bleeding and promote blood clotting and gelation at a wound site (e.g., internal or external laceration, abrasion, incision, puncture, etc.) of a patient. Advantageously, hydrogel formation can be carried out *in situ* by administering the peptide-albumin solution to the patient. Alternatively, the peptide solution alone could be administered to the patient, wherein native (*in vivo*) albumin in the patient's blood and/or blood stream initiates hydrogel formation *in situ* at the wound site.

The hydrogels can also be used as 3-dimensional cell cultures. For example, the cells to be cultured can be mixed with the peptide-albumin solution, and in particular, can be added to the system as part of serum-supplemented cell media. During hydrogelation, the cells become encapsulated within the hydrogel matrix. The hydrogel can then be applied (plated, seeded, etc.) to a cell culture plate or microwell for cell culturing. In one or more embodiments, the hydrogel can be covered with additional cell media to prevent drying out, and the hydrogel containing the cells can be incubated under cell culture conditions (i.e., the appropriate conditions for cell maintenance and/or growth, depending on the cell type). Because of the reversible nature of the hydrogel, the cultured cells can be later isolated from the hydrogel. For example, the hydrogel can be subjected to a mechanical force to disrupt the hydrogel matrix, in combination with diluting the hydrogel (e.g., with additional cell media or other solvent). The resulting liquefied peptide-albumin and cell mixture can then be separated to isolate the cultured cells. This technique is suitable for use with various types of cells including stem cells, cancer cells, primary cells, normal cells, neuron cells, and the like.

Linear, self-assembling peptides are used in forming the hydrogels. Suitable peptides are described in WO 2011/112856. The peptides comprise (consist essentially or even consist of) three segments or regions: a terminal hydrophobic region, a central turning region, and a terminal hydrophilic region. The turning region is positioned between, and preferably directly connected to, the hydrophobic and hydrophilic regions. Thus, the peptides are amphiphilic, with one end segment of the peptide being relatively water loving (aka "hydrophilic"), the other end segment of the peptide being relatively water fearing (aka "hydrophobic"), and the central turning region providing the flexibility for turning and folding. A region is considered "hydrophilic" in the context of the present disclosure, if the region has a greater water affinity than the hydrophobic region of the corresponding peptide. Likewise, a region is considered "hydrophobic" herein, if the region has a greater aversion to water than the respective hydrophilic segment of the corresponding peptide. Accordingly, it will be appreciated that a hydrophobic region may still include one or more hydrophilic amino acid residues, as long as the overall nature of the region is nonetheless more hydrophobic than the corresponding hydrophilic region of the peptide. Similarly, a hydrophilic region may include one or more hydrophobic amino acid residues, as long as the overall nature of the region is nonetheless more hydrophilic than the corresponding hydrophobic region of the peptide. As used herein, it will be appreciated that when referring to amino acids that are present as part of a peptide, the amino acids are actually amino acid residues, regardless of whether "residues" is specifically stated.

The hydrophobic region is preferably elastic and capable of binding the Group I and Group II metals (and particularly calcium). Preferred hydrophobic regions comprise (consist essentially or even consist of) from about 2 to about 15 amino acid residues, preferably from about 4 to about 9 amino acid residues, and more preferably about 5 amino acid residues. The amino acid residues are preferably selected from the group consisting of F, L, I, V, and A. In one or more embodiment, the hydrophobic region is selected from the group consisting of FLIVI (SEQ ID NO:2), GLIVI (SEQ ID NO:5), PLIVI (SEQ ID NO:6), DLIVI (SEQ ID NO:7), VLIVI (SEQ ID NO:8), ILIVI (SEQ ID NO:9), LLIVI (SEQ ID NO:10), ALIVI (SEQ ID NO:11), FGIVI (SEQ ID NO:12), FPIVI (SEQ ID NO:13), FDIVI (SEQ ID NO:14), FVIVI (SEQ ID NO:15), FIIVI (SEQ ID NO:16), FAIVI (SEQ ID NO:17), FLGVI (SEQ ID NO:18), FLPVI (SEQ ID NO:19), FLDVI (SEQ ID NO:20), FLVIV (SEQ ID NO:21), FLAVI (SEQ ID NO:22), FLIGI (SEQ ID NO:23), FLIPI (SEQ ID NO:24), FLIDI (SEQ ID NO:25), FLIII (SEQ ID NO:26), FLILI (SEQ ID NO:27), FLIAI (SEQ ID NO:28), FLIVG (SEQ ID NO:29), FLIVP (SEQ ID NO:30), FLIVD (SEQ ID NO:31), FLIVV (SEQ ID NO:32), FLIVL (SEQ ID NO:33), and FLIVA (SEQ ID NO:34). In one or more embodiment, the hydrophobic region is FLIVI (SEQ ID NO:2).

Preferred hydrophilic regions comprise (consist essentially or even consist of) from about 5 to about 20 amino acid residues, preferably from about 5 to about 10 amino acid residues, and more preferably about 10 amino acid residues. More preferably, the hydrophilic regions comprise amino acid residues selected from the group consisting of G, P, D, V, I, L, and A. In one or more embodiments, the hydrophilic region is selected from the group consisting of [GPXXD]ₙ (SEQ ID NO:35), [GXXPD]ₙ (SEQ ID NO:36), [GXPXD]ₙ (SEQ ID NO:37), and combinations thereof, where n=1-6, and each X is individually selected from the group consisting of G, A, D, R, Q, E, S, T, K and P. In one embodiment, the hydrophilic region comprises, and preferably consists of, in any order, amino acid residues of GPGX¹DGPGX¹D (SEQ ID NO:38), where X¹ is selected from the group consisting of G and A. In another embodiment, the hydrophilic region comprises, and preferably consists of, in order, amino acid residues of GPGX¹DGPGX¹D (SEQ ID NO:38), where X¹ is selected from the group consisting of G and A. In a further embodiment, the hydrophilic region comprises, and preferably consists of, in order or in any order, amino acid residues of GPGX²DGX³X²X²D (SEQ ID NO:39), where each X² is individually selected from the group consisting of A, G, V, I, and L, and X³ is selected from the group consisting of P, A, G, V, I, and L. In yet another embodiment, the hydrophilic region comprises amino acid residues of GPGX²D (SEQ ID NO:40), where X² is defined above. Furthermore, the hydrophilic region could be selected from the group consisting of amino acid residues of [GPGX²DGX³X²X²D]ₙ (SEQ ID NO:39) and [GPGX²D]ₙ (SEQ ID NO:40), where n is from 1 to 10, and more preferably from 1 to 5, and X², and X³ are defined as above.

The most preferred hydrophilic region comprises GPGGDGPGGD (SEQ ID NO:4) (in any order, but preferably in this order), or a fragment or variant having at least about 60% homology to this sequence, and retaining the functional characteristics thereof. More preferably, the % homology to this sequence is at least about 80% and even more preferably at least about 90%, and retaining the functional characteristics thereof.

For a functional hydrogel-forming peptide in this design, the hydrophobic region is not directly connected to the hydrophilic region, but includes a turning region in-between. The turning region provides structural flexibility which allows the potentially charged side-chains of the hydrophilic residues to come in proximity and help the segregation of hydrophobic and non-hydrophobic side-chains. Thus, it will be appreciated that the central region is considered a "turning" region in the context of the present disclosure because it is comprised of amino acids with small side chains allowing for flexibility and "turning" in that region of the peptide.

Preferred turning regions comprise from about 1 to about 12 amino acid residues, preferably from about 4 to about 8 amino acid residues, and preferably 4 amino acid residues. The turning region of the inventive peptides preferably comprises amino acids residues selected from the group consisting of G, L, I, V, A, S, and T. In one or more embodiments, the turning region is selected from the group consisting of G, GG, GGG, GGGG (SEQ ID NO:41), GSX⁴X⁴ (SEQ ID NO:42), X⁴GSX⁴ (SEQ ID NO:43), X⁴X⁴GS (SEQ ID NO:44), SGX⁴X⁴ (SEQ ID NO:45), X⁴SGX⁴ (SEQ ID NO:46), X⁴X⁴SG (SEQ ID NO:47), GX⁴SX⁴ (SEQ ID NO:48), X⁴GX⁴S (SEQ ID NO:49), SX⁴GX⁴ (SEQ ID NO:50), X⁴SX⁴G (SEQ ID NO:51), GX⁴X⁴S (SEQ ID NO:52), and SX⁴X⁴G (SEQ ID NO:54), where each X⁴ is individually selected from the group consisting of G, I, V, A, L, S (and where S could be replaced by T in all sequences listed). One preferred turning region comprises, and preferably consists of, amino acid residues of X⁵SX⁶X⁶ (SEQ ID NO:54), in any order (even more preferably in this order), where X⁵ is selected from the group consisting of G, I, and V, with G being particularly preferred, and each X⁶ is individually selected from the group consisting of G, I, V, A, and L, with I being particularly preferred. Preferably, at least one of X⁵ or X⁶ is G, with it being particularly preferred that at least X⁵ is G. In one embodiment, S of X⁵SX⁶X⁶ (SEQ ID NO:54) could be replaced with T. In one or more embodiment, the turning region is GSII (SEQ ID NO:3).

The peptides are preferably short peptides. That is, it is preferred that the peptides have less than about 30 amino acid residues, more preferably less than about 20 amino acid residues, and even more preferably 19 amino acid residues. The most preferred peptide according to the invention comprises (consists essentially or even consist of) the amino acid sequence FLIVIGSIIGPGGDGPGGD (SEQ ID NO:1), or a fragment or variant thereof having at least about 60% homology to this sequence, more preferably at least about 80% homology to this sequence, and even more preferably at least about 90% homology to this sequence, and retaining the functional characteristics thereof.

Finally, the peptides will have a weight average molecular weight of from about 600 Da to about 4,500 Da, more preferably from about 1,000 Da to about 3,000 Da, and more preferably about 1,740 Da.

The peptides can be prepared by microwave synthesizer, microbiosynthesis, fermentation, or genetic engineering technologies. A preferred method involves combining two native sequences from an elastic segment of spider silk and a trans-membrane segment of human muscle L-type calcium channel. More specifically, the most preferred hydrophilic region, GPGGDGPGGD (SEQ ID NO:4), is preferably designed from a β-spiral motif of spider flagelliform silk protein, while the most preferred hydrophobic region FLIVI (SEQ DI NO:2) and turning region GSII (SEQ ID NO:3), are derived from the third trans-membrane segment of subunit IV in the dihydropyridine sensitive human muscle L-type calcium channel. After precipitation and washing, the peptides can be freeze-dried (lyophilized) for storage until use.

Additional advantages of the various embodiments of the invention will be apparent to those skilled in the art upon review of the disclosure herein and the working examples below. It will be appreciated that the various embodiments described herein are not necessarily mutually exclusive unless otherwise indicated herein. For example, a feature described or depicted in one embodiment may also be included in other embodiments, but is not necessarily included. Thus, the present invention encompasses a variety of combinations and/or integrations of the specific embodiments described herein.

As used herein, the phrase "and/or," when used in a list of two or more items, means that any one of the listed items can be employed by itself or any combination of two or more of the listed items can be employed. For example, if a composition is described as containing or excluding components A, B, and/or C, the composition can contain or exclude A alone; B alone; C alone; A and B in combination; A and C in combination; B and C in combination; or A, B, and C in combination.

The present description also uses numerical ranges to quantify certain parameters relating to various embodiments of the invention. It should be understood that when numerical ranges are provided, such ranges are to be construed as providing literal support for claim limitations that only recite the lower value of the range as well as claim limitations that only recite the upper value of the range. For example, a disclosed numerical range of about 10 to about 100 provides literal support for a claim reciting "greater than about 10" (with no upper bounds) and a claim reciting "less than about 100" (with no lower bounds).

### EXAMPLES

The following examples set forth methods in accordance with the invention. It is to be understood, however, that these examples are provided by way of illustration and nothing therein should be taken as a limitation upon the overall scope of the invention.

### EXAMPLE I

### Peptide-albumin hydrogel formation and properties

### Introduction

Because of its distinct three-dimensional network, peptide hydrogel not only provides an *in vitro* environment that mimics the extracellular matrix conditions for 3D cell culture but also acts as an auxiliary carrier for targeted drug or gene delivery and biomolecular controlled release. With the rapid development of peptide hydrogels for biomedical applications, the mild method to trigger the peptide solution into hydrogel has attracted more attentions to apply these materials. In our recent study, we found that the h9e peptides could spontaneously organize into an injectable hydrogel material under the trigger of Bovine Serum Albumin (BSA) or Human Serum Albumin (HSA) without adding any other metal ions or adjusting environmental pH or temperature. This is the first time a rationally designed peptide has been found to self-assemble into hydrogel through binding with albumin molecules. The objective of this study is to understand how albumin triggers h9e peptide self-assembly into hydrogel. The storage modulus of hydrogel as well as the hydrogel forming rate are affected by peptide and albumin concentration. Because albumin is the most abundant protein in human serum and is considered one of the most important proteins for molecular transportation, pH balance, and maintaining osmotic pressure, hydrogel formed through albumin binding has great potential for a wide range of applications.

### Methods and Materials

### 1. Peptide Synthesis and Hydrogel Preparation

Peptides were synthesized on a CEM Liberty microwave peptide synthesizer (CEM Corporation, Matthews, NC) based on the automated base-labile 9-fluorenylmethoxycarbonyl (Fmoc) strategy with Fmoc-protected amino acids (EMD Biosciences, San Diego, CA). Peptides were cleaved with 95% trifluoroacetic acid (Sigma-Aldrich, Milwaukee, WI), 2.5% triisopropylsilane (Sigma), and 2.5% deionized water. The crude peptides were washed three times with anhydrous ether (Fisher Biotech, Fair Lawn, NJ). After that, peptides were dissolved in acetonitrile and distilled (DI) water (50/50 v/v). The peptide solution was frozen in a -80 °C refrigerator overnight and then was freeze-dried for 48 hours by using the Labconco freeze dry system (Labconco, Kansas City, MO). The pH value of the peptide in water solution was 3.6. Molecular weight of the synthesized peptide was confirmed by matrix-assisted laser desorption/ionization time-of-flight mass spectroscopy on an Ultraflex II instrument (Bruker Daltronics, Billerica, MA). The peptide purity was confirmed by a Beckman System Gold high performance liquid chromatography (HPLC, Beckman Coulter, Inc., Fullerton, CA) on a phenomenex synergi 4µ Hydro-RP column (Phenomenex, Inc., Torance, CA) with the following gradient: 10-90% B in 20 min (A: 99.9% H2O, 0.1% TFA; B: 90% acetonitrile, 9.9% H2O, 0.1% TFA).

For albumin triggered hydrogel formation, 10 mM (1.74 wt %) peptide was first dissolved in 100 mM NaHCO₃ solution. BSA and HSA were dissolved in water with 5 wt % concentration. The peptide solution and albumin solution were then mixed at different ratios for final mixtures of 1, 2, 3 mM h9e peptide with albumin from 0.1 to 5 wt %.

### 2. Transmission Electron Microscopy (TEM)

Peptide solutions were prepared by a negative strain method in which 20 µl peptide solution was placed on Formvar/carbon-coated 200-mesh copper grids (Electron Microscopy Sciences, Fort Washington, PA) for 1 min. The extra solution was removed and the TEM grids were floated on the top surface of 2% (w/v) uranyl acetate (Ladd Research Industries, Inc., Burlington, VT) for 60 s at ambient conditions. The TEM grids were removed and dried before imaging. The samples were observed with a CM100 TEM (FEI Company, Hillsboro, OR) at 100 kv.

### 3. Rheology

The storage (G') and loss (G") moduli of h9e hydrogels with different peptide/albumin ratios were measured on a rheometer system C-VOR 150 (Malvern instruments, Malvern, Worcestershire WR141XZ, United Kingdom) with a 20 mm diameter parallel plate test system. All rheological tests were carried out at 37 °C. To determine the hydrogel forming rate, the peptide and albumin mixture was placed on the measuring system immediately after preparation and tested by a single frequency (1 Hz) method with steady shear strain (1%) for 1 hour

### 4. Hydrogel Properties

We found serum in the cell medium was an important factor to help hydrogel formation: without any additional chemical or environmental adjustment, h9e peptide solution could be directly added into Minimum Essential Medium (MEM) with 10% serum and transformed into a self-supporting hydrogel matrix within 1 min. However, using MEM without serum did not stimulate this solution to hydrogel transitions effectively. This phenomenon was further exploited in other cell culture medium like Dulbecco's modified Eagle's medium (DMEM), Roswell Park Memorial Institute medium (RPMI), and Leibovitz medium (L-15). Because serum is an extremely complex composition, determining the major factors that triggered hydrogel formation was difficult. After screening the abundant serum compounds, we concentrated on one of the most abundant proteins in serum, albumin, which contains charged groups and several surface binding domains. Interestingly, the hydrogel formation of h9e peptide in a serum-free N2B27 supplemented 2i medium with 1 wt% albumin confirmed our hypothesis.

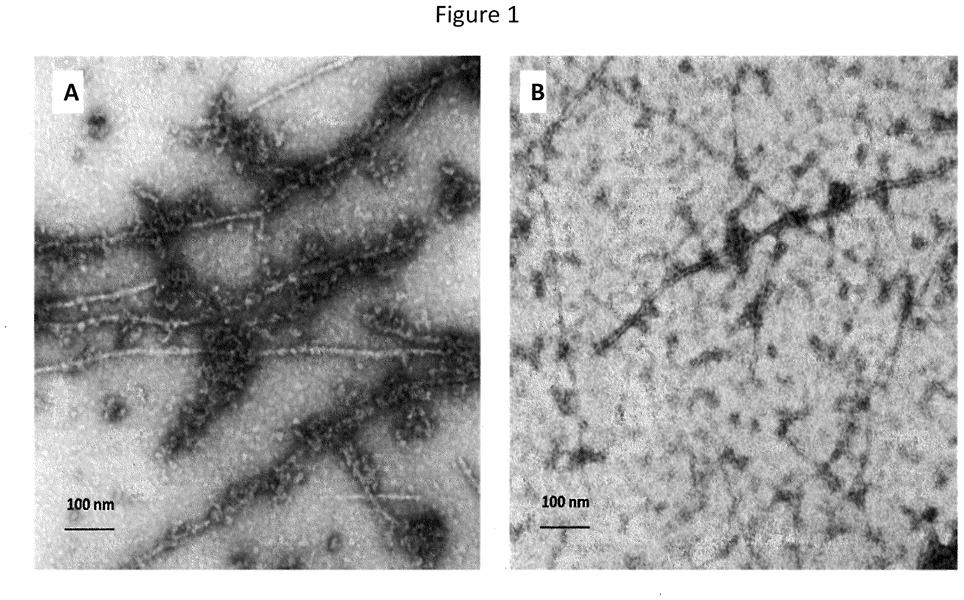

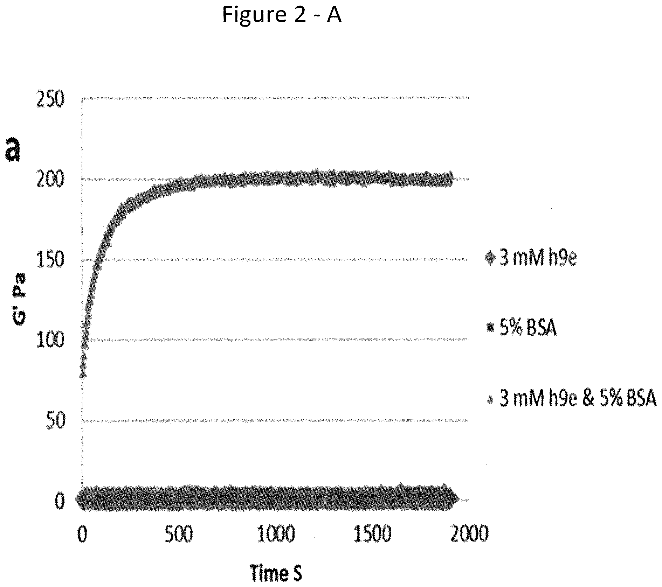

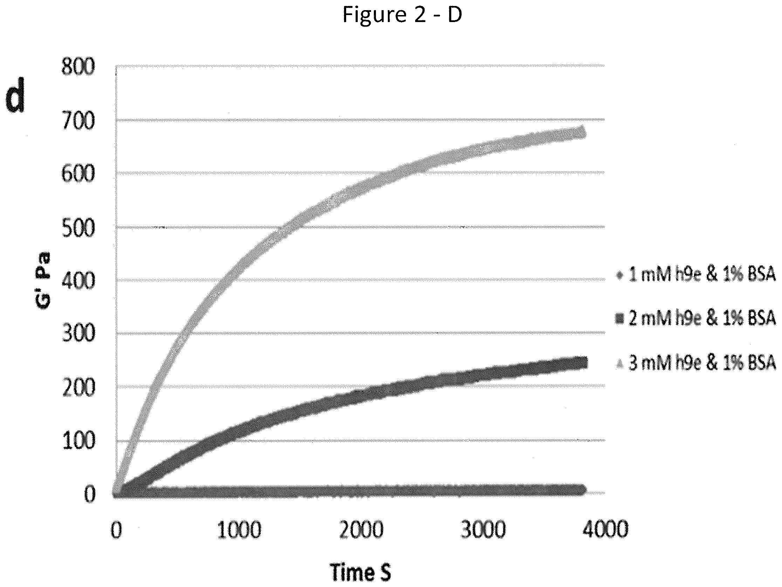

To demonstrate this albumin-induced peptide self-assembly pathway, we prepared h9e/BSA and h9e/HSA mixtures and observed them under TEM. Figure 1 shows that peptide nanofibers bind on the surface of both albumins. Figure 1a shows the BSA attached to the peptide nanofibers and settled along the fiber growth direction. Similarly, Figure 1b shows peptide nanofibers touched the surfaces of HSA from one point to another. These visible data confirm the interactions of h9e peptide and albumin molecules. In addition, the hydrogel-forming process was monitored by rheological testing. Figure 2a presents the storage modulus (G') change of 3 mM h9e peptide solution right after mixing with 5% BSA. In contrast to a 3 mM h9e solution or 5% BSA solution, which performed as Newtonian liquid during the 30 min test, the mechanical strength of the h9e/BSA mixture kept increasing within the first 500 s and reached a stable G' around 200 Pa.

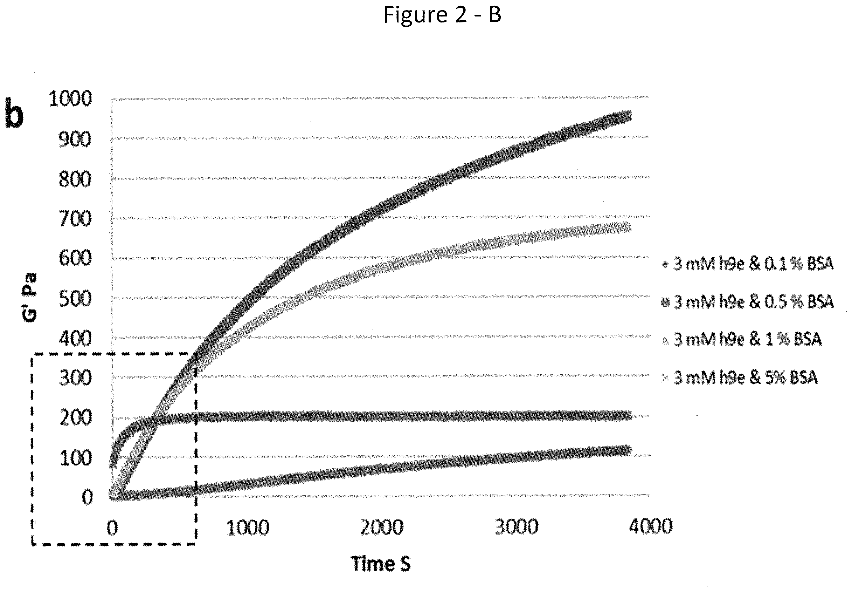

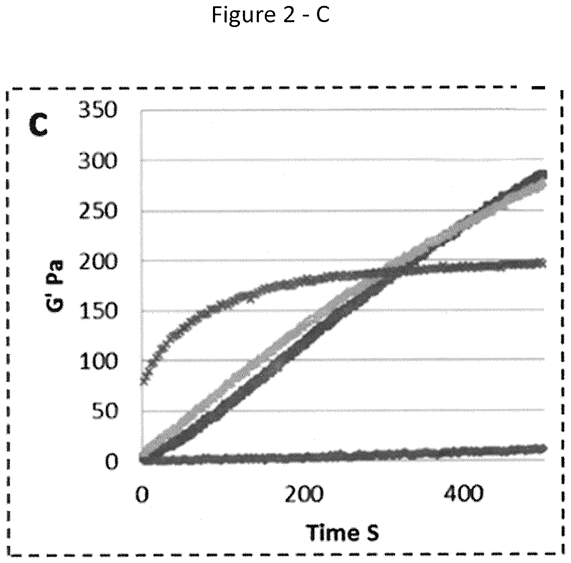

The relationship of h9e peptide and BSA was further explored by testing the mechanical strength and hydrogel forming rate of 3 mM h9e with BSA ranging from 0.1 wt % to 5 wt %. Figure 2b shows an interesting phenomenon: the finial strength of the h9e/BSA hydrogel with the same peptide concentration does not correspond to the BSA concentration. For example, after 2 hours of testing, the G' of peptide with 0.1 wt % BSA is about 110 Pa. The G' increased to over 900 Pa for peptide hydrogel with 0.5 wt % BSA but reduced in gradient to 680 Pa and 200 Pa for peptide with 1 wt% and 5 wt % BSA, respectively. On the other hand, the enlarged screen (Figure 2c) of the first 500 s data shows the increasing rate of G' is consistent with the BSA concentration, which indicates that albumin helps peptide molecules self-assemble into hydrogel; while, as we suggested in our previous study that kinetics is a key factor for peptide self-assembly, relatively lower BSA concentration would allow slower assembling rate for peptide and lead to better nanostructural arrangement for stronger mechanical strength.

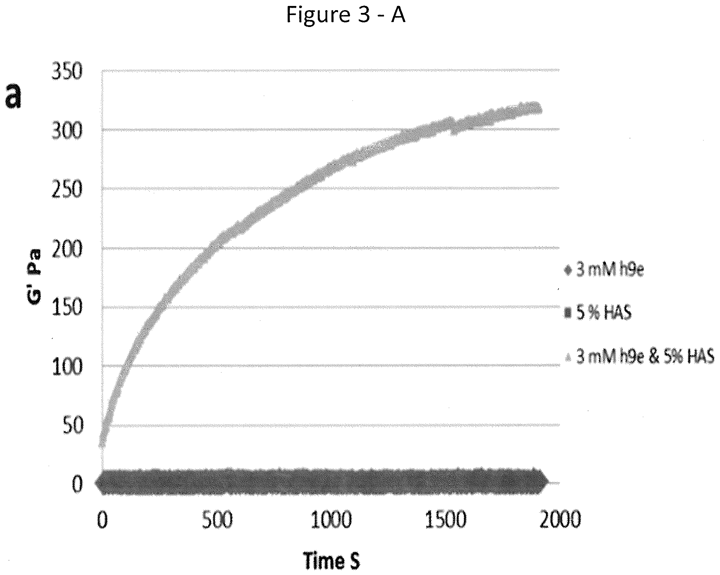

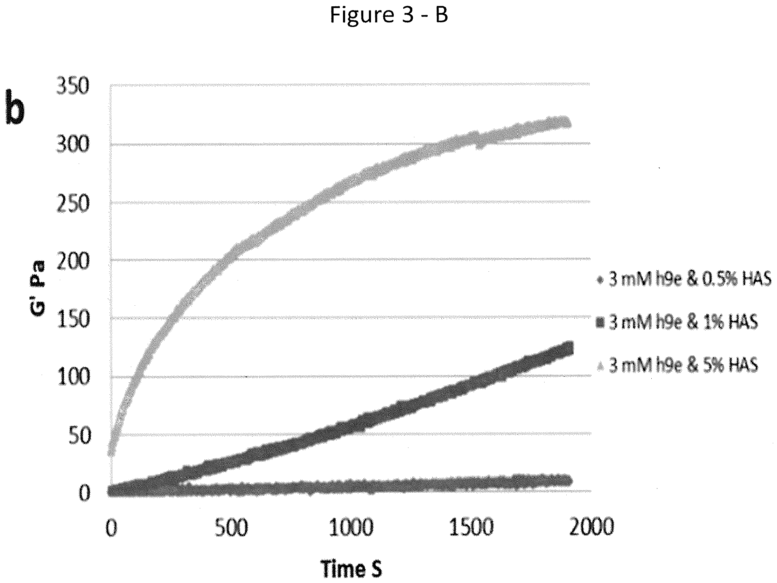

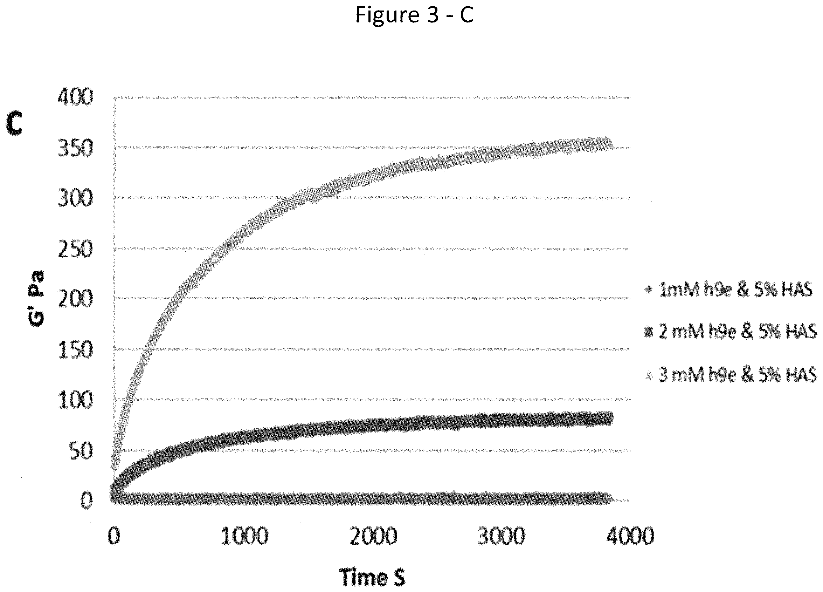

The mixtures of different peptide concentration (1-3 Mm) with constant BSA concentration (1 wt%) were also studied. Higher peptide concentration leads to stronger and faster hydrogel growth. Solution of 1 mM peptide with 1 wt % BSA performed like a Newtonian liquid after a 2 hour test suggesting an important peptide and BSA concentration may exist for this hydrogel forming process. Similar phenomenon has been found in h9e/HSA mixture (Figure 3): hydrogel was formed when 3 mM h9e peptide was mixed with 5 wt % HSA (Figure 3a), and the hydrogel strength and forming rate were more sensitive to HSA concentration compared to h9e/BSA mixture. Hydrogel did not form when peptide concentration was 1 mM or HSA concentration was 0.5 wt %, which again suggested the requirement of the critical concentration of both compounds for h9e/HSA hydrogel formation (Figure 3b, c).

### EXAMPLE II

### Hydrogel properties of peptide h9e in various cell mediums

### Background

Two-dimensional (2D) substrates, such as tissue culture polystyrene and the surface of tissue analogs, make an enormous contribution to modern in vitro cell studies; however, traditional 2D platforms cannot accurately mimic the complex 3D architecture of the extracellular matrix (ECM) where native cells reside. In 2D culture, the monolayer cells experience homogenous concentration of nutrients and growth factors which induce unnatural cell environments and cell-cell interactions, yielding a flat and stretched morphology. Recent studies have shown that the morphological differences of cells cultured in 2D and 3D can exhibit several striking differences in subtle cellular processes such as proliferation, apoptosis, differentiation, gene expression, migration, and drug sensitivities. On the other hand, the biological *in vivo* 3D systems, such as animal models, are expensive and time-consuming. Therefore, advanced *in vitro* 3D model systems are needed to fill the gap between the inaccurate 2D systems and the animal models to mimic the complexity of the ECM and the physiological relevance of an in vivo biological system.

In the last few decades, hydrogel scaffolds, cross-linked networks that possess high water contents, have attracted more and more attention in an attempt to mimic *in vivo* conditions for cell culture. The reticulated structure of cross-linked polymer chains with high water contents introduces a number of desirable cellular microenvironment characteristics: 3D spatial support for cell growth; porosities for cell migration; and facile transportation of oxygen, nutrients, waste, and soluble factors. Hydrogels can be formed from a range of natural sources and synthetic materials. Natural gels derived from ECM components and other biological sources such as collagen, fibrin, hyaluronic acid, chitosan, and alginate are biocompatible and inherit bioactivities that promote cell survival, proliferation, differentiation, and cellular function of many cell types. However, natural hydrogels have varying biochemical presentations and material properties that are difficult to control, which increases the risk and complexity of cellular study in this culture system. On the other hand, synthetic gels are highly reproducible with consistent composition and predictable manipulation of properties. However, synthetic polymers such as polyactide and polyglycolide have too large fiber diameter and porous size, which present poor scaffold structure and mechanical properties to accurately mimic the full complexity of the natural environment of cell growth. With the rapid development of rationally designed peptides as biological materials, peptide based hydrogel was considered as one of the most promising materials for 3D cell culture because of its amino acid composition and the structural and mechanical similarity to natural ECM.

In addition, for *in vitro* 3D cell culture, cell encapsulation and isolation are two critical steps to introduce 3D spatial support for cell growth and to recover embedded cells from the scaffold matrix for downstream studies respectively. For a convenient, effective, and safe encapsulation, cells should be added simultaneously with the initialization of hydrogelation. Therefore, mild and cyto-compatible hydrogel-forming conditions are preferred, to ensure that cells survive comfortably during gel formation. However, the sol-gel transformation of currently used peptide/protein hydrogels (i.e., puramatrix gel, hydromatix peptide hydrogel, and matrigel) is triggered by adjusting pH or temperature (Table 1).

**Table 1. Comparison of material properties, cell encapsulation/recovery, and handling of different 3D cell culture hydrogels**

| **Characteristi cs** | **h9e** | **Puramatrix gel (BD Biosciences) [a]** | **Matrigel (BD Biosciences) [b]** | **Alginate hydrogel (ALgimatrix) [c]** |
|---|---|---|---|---|
| Material | Peptide (19 unit) | peptide (16 unit) | Reconstituted basement membrane extracted from EHS mouse tumor | Polysaccharides (dried sponge) |
| Porosity | 50-200 nm | 50-200 nm | 50-400 nm | 50-200 µm |
| Solution pH | Neutral | Acidic pH 3 | Various during the storage (acidic to physiological pH) | Dry |
| Gel trigger | Hydrogel could be triggered by directly mixing cell medium containing serum or albumin or solution containing Ca2+, Na+ or albumin, or (no pH or temperature adjustment) | Starts gel at pH higher than 4.5-5 (change medium at least 3 steps within first 30 min to equilibrate the sample to physiological pH). | Starts gel at temperature higher than 10 °C | Add gel firming buffer containing Ca2+ |
| Cell encapsulation | Directly mix (pipette), cells suspended in cell medium before the peptide solution is added in a relaxed working environment. Cells are surrounded by medium and nanofibrils network during hydrogelation | Directly mix (pipette, has to be very fast, within 1 min, to shorten the contact time of cell with acidic peptide solution); cells is isolated from medium and prepared in 10% sucrose solution before peptide solution is added | Directly mix with chilled pipette (need to chill everything before experiment because temperature is the trigger for gelation | Immediately centrifuge after the firming buffer added (for better cell distribution) |
| Cell recovery | Pipette, dilute the hydrogel with cell medium 1:15 folds and centrifuge | Pipette to disturb the gel structure and centrifuge | Add cell recovery solution or lowing temperature or centrifugation to disrupt the gel matrix | Add dissolving buffer |

The undesirable low pH or cold temperature of the pre-gel solutions may cause cell death when they are directly mixed. Hydrogel preparation procedures become complex when changing cell medium for pH balance or chilling experimental tools are required (Table 1). Cells kept in sucrose solution or a gel-forming buffer struggle with lack of nutrients up to several hours during gel formation before cell medium can be added. Moreover, isolating cells from the hydrogel matrix is another challenge for 3D cell culture. In most cases, changing the environmental factors back to extreme conditions or adding undesirable buffer for hydrogel degradation are required to initialize the gel-sol transformation before cells can be separated out (Table 1). This process threatens the survival of cultured cells and may cause the failure of the whole downstream studies. Therefore, it is necessary to develop a hydrogel which not only presents a convenient and effective process for cell encapsulation, but also provides an easy and safe cell isolation for further cell physiological and pathophysiological studies.

### Materials and Methods

### A. Peptide-medium hydrogels in cancer cell mediums

A solution of h9e was prepared at neutral pH (∼7) and mixed with Minimum Essential Medium (MEM, with 10% FBS (source of albumin)) at room temperature. After mixing, h9e peptides self-assemble into a hydrogel matrix with a final peptide concentration as low as 1 mM (0.17%). Without introducing any additional gel forming buffer or adjusting environmental pH or temperature, this peptide provides a convenient and mild hydrogel forming process and allows cells to be surrounded by their culture medium during cell encapsulation (Table 1). More interestingly, the mechanical strength of this hydrogel matrix exhibits special deformability and reassembly capability, which allow the gel-sol transformation through repeated pipetting.

A breast cancer cell line, MCF-7, was selected as a model to grow in 3D in the h9e-MEM hydrogels. Studies of cell morphology, viability and proliferation showed that cells exhibited 3D cyto-architecture in the hydrogel matrix and kept high bioactivities for further studies after isolation.

### 1. Peptide synthesis and hydrogelation

The h9e peptide was synthesized according to a previously published protocol. Briefly, peptides were synthesized on an automated CEM Liberty microwave peptide synthesizer (CEM Corporation, Matthews, NC) according to the base-labile 9-fluorenylmethoxycarbonyl (Fmoc) strategy with Rink amide resin and Fmoc-protected amino acids. After final N-terminal Fmoc group deprotection, the resin-bound peptides were side-chain-deprotected and cleaved using TFA/TIS/water (95/2.5/2.5 v/v). Peptides were precipitated and washed three times with anhydrous ether, dissolved in acetonitrile and deionized water (50/50 v/v), then freeze-dried. Molecular weight and purity of the synthesized peptides were confirmed by matrix-assisted laser desorption/ionization time-of-flight mass spectroscopy and high-performance liquid chromatography.

Lyophilized peptide was added to 100 mM sodium bicarbonate and completely dissolved by magnetic stirring for 3 hours with a final peptide concentration of 10 mM. For hydrogelation, peptide solution was added into MEM with 10% FBS and the mixture was hand-shaken for about 10 seconds. The peptide hydrogel formed within 15 minutes at room temperature with final peptide concentrations of 1, 2, and 3 mM.

### 2. Rheological tests

The storage and loss moduli (G' and G", respectively) of h9e hydrogels were determined on a C-VOR 150 rheometer system (Malvern instruments, Malvern, Worcestershire WR141XZ, United Kingdom) with a 20-mm diameter parallel plate geometry and 500 µm gap size. To mimic cell physiological conditions, all rheological tests were performed at 37 °C unless otherwise specified. The peptide and MEM mixture was placed on the measuring system immediately after mixing for a gel-forming rate test. Single frequency (1 Hz) and steady shear strain (1%) were selected for a 1hour test. To determine the hydrogel reassembly capability, the peptide and MEM mixture was incubated at room temperature overnight for hydrogelation, then transferred to a lower measuring plate for a 10 minute, single-frequency test (1 Hz, 1% strain) for stabilization. The hydrogel was broken using 1 Hz frequency and 500% shear strain for 1 minute. Resetting the instrument parameters took 1 minute, and the hydrogel moduli during the reassembly period were measured under 1 Hz frequency and 1% shear strain for 1 hour. The amplitude sweep test (strain from 1 to 500%, 1Hz frequency) was conducted multiple times to determine hydrogel reassembly capability after each time it was destroyed. Four testing cycles were applied in this measurement and the hydrogel recovery time between every two cycles was 1, 5, and 10 minutes. Furthermore, to test the response to different environmental temperatures, the peptide hydrogel was measured under a temperature profile test with steady oscillatory frequency (1 Hz) and strain (1%). The temperature was adjusted from 4 °C to 50 °C for two testing cycles. For each cycle, the instrument's heating or cooling processes took 5 minutes, then another 5 minutes to arrive at the setting temperature (4 °C or 50 °C). To determine the G' and G" of hydrogel during cell isolation, 3 mM peptide hydrogel was diluted 15 times with MEM. After thorough mixing, the diluted solution was tested under 1 Hz frequency and 1% shear strain at 4 °C for 1 hour.

### 3. Scanning electron microscopy (SEM)

The nanofiber network of hydrogel scaffolds, as well as surface characteristics of the 3D cultured cells, were observed under SEM. The hydrogel samples were dehydrated with increasing concentrations of ethanol from 50% (v/v) to 100% (v/v) at 5% per step and 15 minutes for each step. The ethanol was then removed by a critical point dryer (Samdri-790B, Tousimis Research Corp., Rockville, MD). The hydrogel samples with cells were fixed in a 2% paraformaldehyde and 2% glutaraldehyde mixture for 30 minutes before dehydration and critical point drying. Samples were then sputter-coated (Desk II Sputter/etch Unit, Denton Vacuum, Moorestown, NJ) 3 times (12 seconds each time) with 100% Pt. The SEM observation was carried out with an FEI, Nova NanoSEM 430 (Hillsboro, ON) at 5 kV and through a lens detector.

### Results and Discussion

### 1. Peptide hydrogelation in MEM

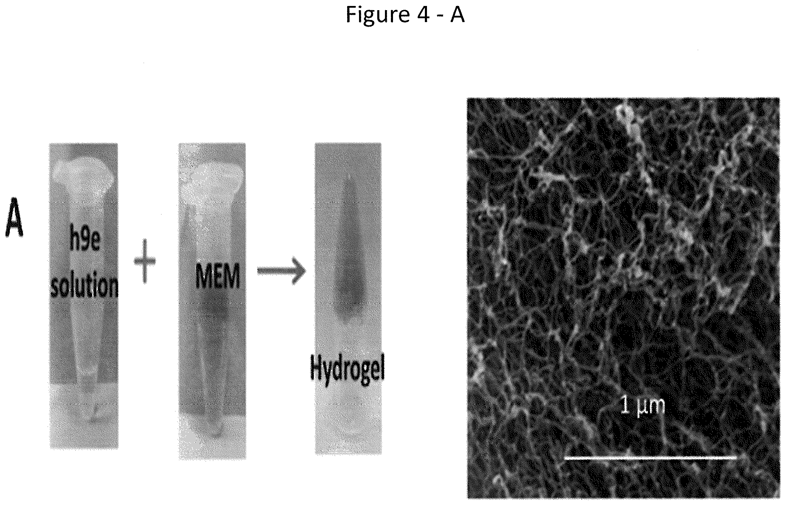

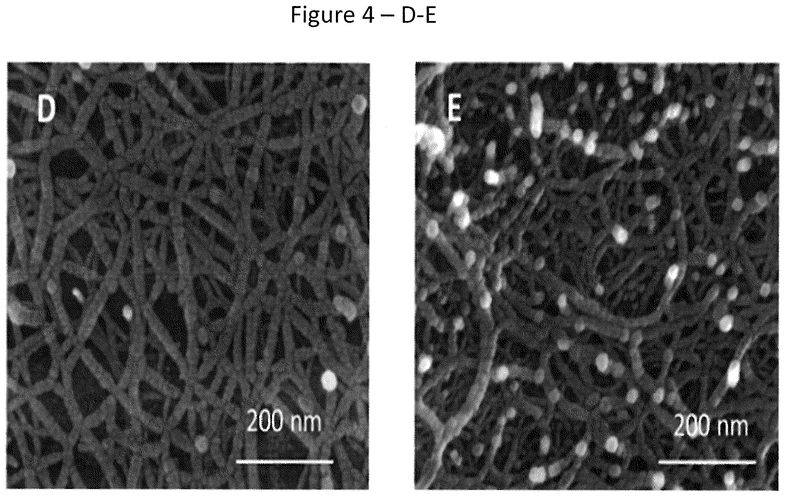

To initiate gel-formation, 100 (µl of 10 mM h9e peptide solution (pH 7-8) was added to 900 µL Minimum Essential Media (MEM, with 10% FBS (source of albumin)) to form 1 mL mixture with 1mM (0.17% w/v) peptide concentration (Figure 4A). The nanoscale morphology of the hydrogel matrix is presented by the SEM image (Figure 4A). Peptide hydrogelation induced directly by mixing neutral pH peptide solution with MEM not only avoids the complex chemical gel cross-linking processes, but also utilize a medium commonly used in biological and medical research, providing a physiological condition to cell encapsulation.

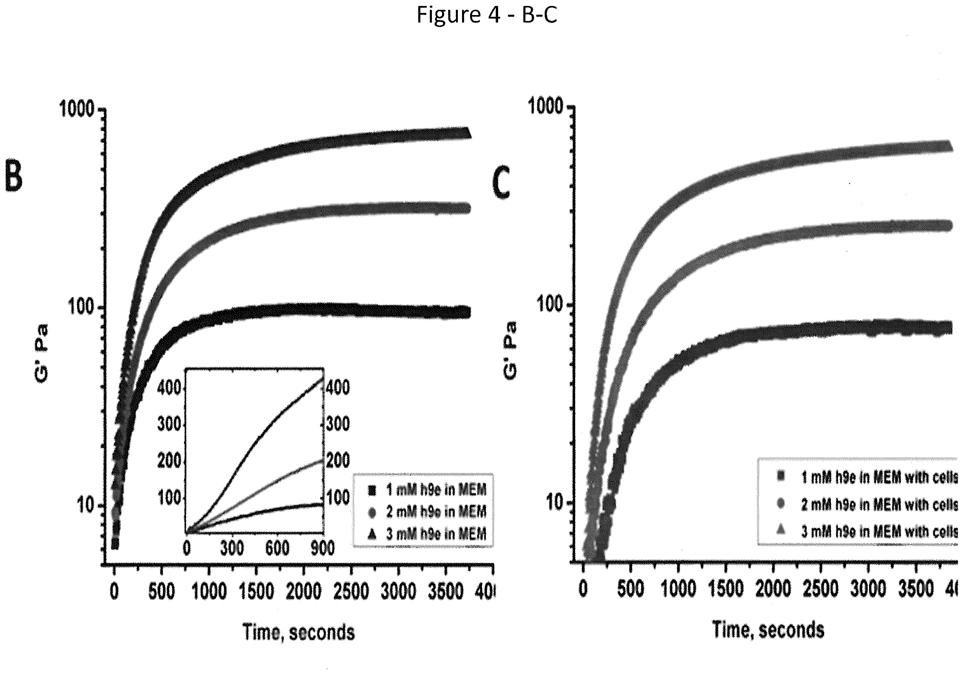

Direct loading of drugs, proteins, or cells during gel formation is one of the most convenient and effective ways for encapsulation. To ensure homogenous distribution of embedded molecules, peptides should assemble as a nanofiber network in a relatively short period with reasonable strength to hold the suspended molecules before their precipitation. To determine the peptide gel-formation rate, we prepared hydrogels with three concentrations, 1 mM (0.17% w/v), 2 mM (0.34% w/v), and 3 mM (0.51% w/v), in MEM. The storage modulus of the solution was measured at 37 °C immediately after thorough mixing. Figure 4B shows the h9e peptide hydrogel formations with stable storage modules around 100, 400, and 700 Pa, respectively. The gel-formation rates increase with peptide concentrations (inset of Figure 4B), and all three hydrogels reached a self-supporting strength (close or above 100 Pa) within 15 minutes. SEM images (Figure 4C, D) indicate that the hydrogel architecture is built by entanglement of 20 nm width nanofibers; however, the lower-concentration hydrogel (1 mM, Figure 4C) shows a relatively looser matrix structure compared with the compact structure of the higher-concentration hydrogel (3 mM Figure 4D). This visual evidence further supports the strength differences of different concentration hydrogels.

### 2. Dynamic rheological study of h9e hydrogel

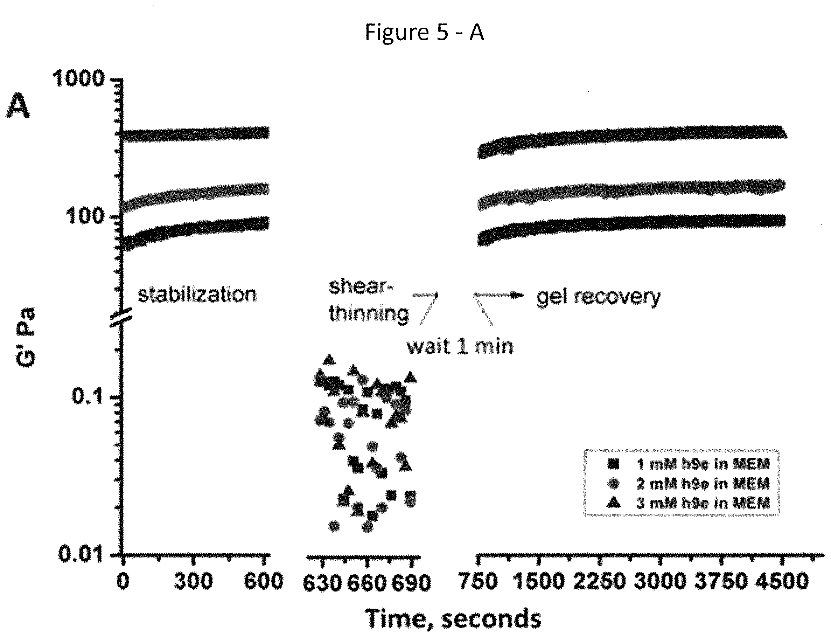

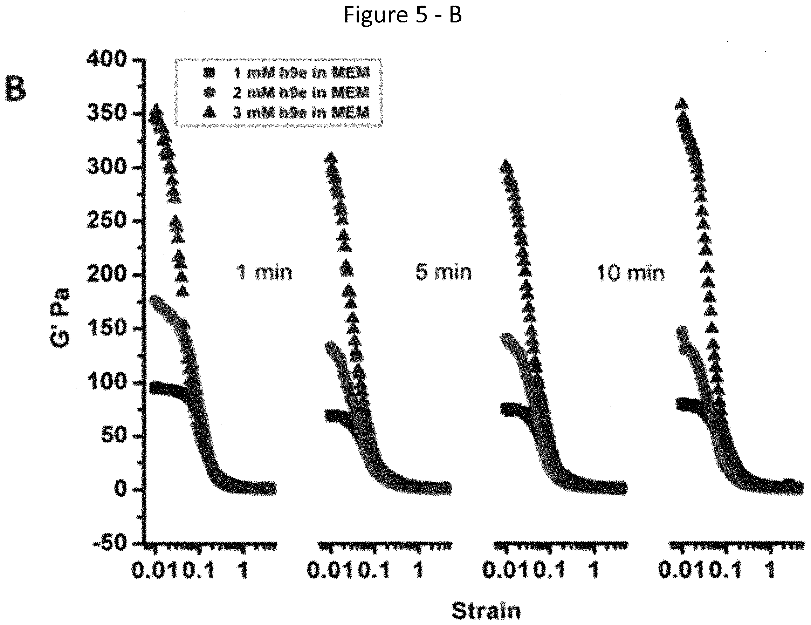

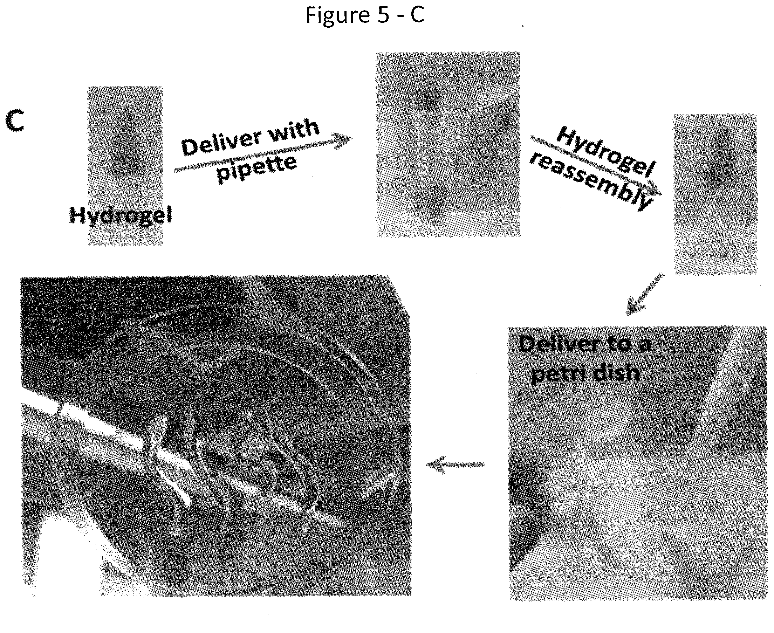

The deformability and reassembly ability of MEM-induced h9e hydrogel were assessed by a dynamic rheological test: 1-3 mM peptide hydrogels were stored at room temperature overnight, then transferred to a measuring system and stabilized for 10 minutes. By shear-thinning at 500% strain for 1 minute, all three hydrogels were converted to liquid state, showing a G' lower than 0.2 Pa (Figure 5A). After shear-thinning stopped, instrument parameters were reset after 1-min waiting time and the hydrogel recovery was monitored using 1% shear strain for 1 hour. The data in Figure 5A shows the G' of hydrogel recovery during this 1 hour test. To determine whether the hydrogel could maintain this reassembly capability even after shear-thinning many times, the hydrogel was measured under an amplitude sweep test conducted multiple times. Four testing cycles were applied in this measurement and shear strain was increased from 1% to 500% within 5 minutes for each cycles. After that, for hydrogel recovery, the waiting time of 1, 5, and 10 minutes were applied, respectively. Figure 5B suggests that although the hydrogel architecture was completely broken into liquid form at the end of each cycles, quick reassembly persisted even after shear-thinning multiple times. The results also show that the percentage of recovery G' increased with waiting time and that the hydrogel reassembly rate related to hydrogel concentrations. For example, with a waiting time of 1 min, about 73%, 76%, and 88% of the gel strengths were recovered for 1, 2, and 3 mM hydrogel, respectively, but after three shear-thinning cycles and 10 min waiting, 83% (1 mM), 84% (2 mM), and 100% (3 mM) of the gel strength was recovered (Figure 5B). The higher reassembly rate is most likely caused by the more compact matrix structure of hydrogel due to higher peptide concentration (3 mM) (Figure 4D). In the solution with high peptide concentration, some noncovalent gel network cross-links remain intact and the broken nanofiber groups are close to each other, making rebuilding the cross-links easy. Based on these rheological properties, the MEM-induced h9e hydrogel could be delivered via pipette multiple times without permanently destroying the hydrogel architecture (Figure 5C). This special shear-thinning and recovery property of the hydrogel also provides an alternative method for cell isolation from hydrogel matrix through a mechanical shearing and dilution.

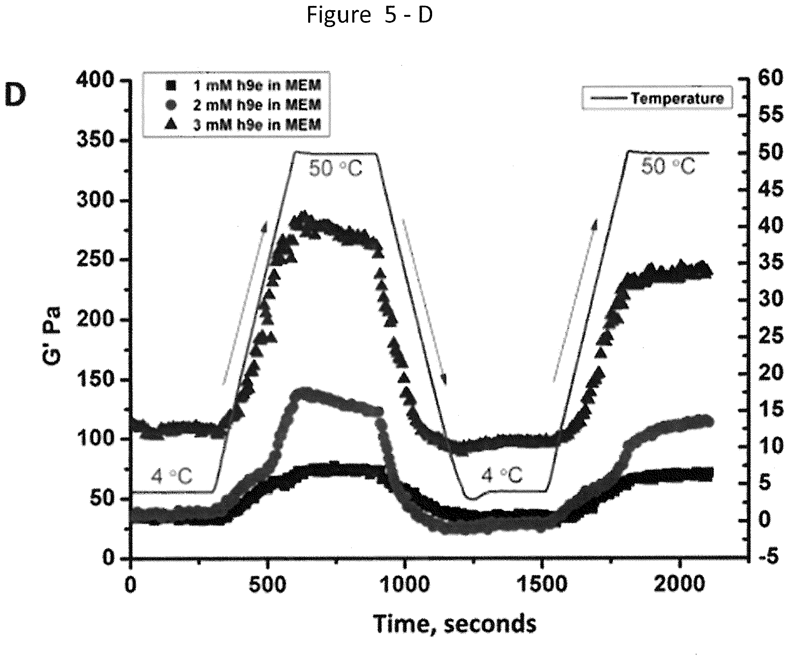

For biological study, temperatures between 4 °C and 37 °C are commonly applied for many standard operational procedures *in vitro;* therefore, understanding the response of the hydrogel materials to these temperature variations has a large impact on their practical applications. The rheological temperature profile test was performed to address this challenge. The temperature was adjusted from 4 °C to 50 °C for two testing cycles. Figure 5D shows that the G' of hydrogels moves along with temperature and performs 2-3 times higher at 50 °C than that at 4 °C. This thermal response is reversible according to the hydrogel heating and cooling cycles (Figure 5D). The results provide support for using this peptide hydrogel as a matrix for 3D cell culture: the hydrogel matrix is stiffened for cell encapsulation when it remains at 37 °C, but is weakened at 4 °C for cell isolation using standard centrifuge method.

### B. Peptide-medium hydrogels in stem cell mediums

Two different types of stem cell mediums were selected for this test. One is a low glucose DMEM medium with 2 % (v/v) Fetal Bovine Serum (FBS) for Mesenchymal Stromal Cell (MSC medium). The other one is a N2B27 supplemented serum-free 2i medium with 0.5 % (v/v) Bovine Serum Albumin (BSA) for rat Embryonic Stem Cell (ESC, 2i medium). The hydrogel formation process was monitored under a time sweep rheological test for 1 hour. The special shear-thinning and self-healing property of h9e peptide hydrogel was also tested. After hydrogel was stable, more cell medium was added to dilute the hydrogel 20 fold; the data of storage modulus (G') was used to demonstrate the cell recovery capability of this hydrogel system. In addition, the stability of hydrogel as well as the peptide solution before the hydrogel formation was also tested.

### 1. Peptide synthesis and hydrogelation

The h9e peptide was synthesized according to the method mentioned above. Lyophilized peptide was added to 100 mM sodium bicarbonate and completely dissolved by magnetic stirring for 3 hours with a final peptide concentration of 10 mM. For hydrogelation, peptide solution was added into MSC medium or 2i medium and the mixture was hand-shaken for about 10 seconds or gently pipette mixed 3 times. The final peptide concentrations were 1, 2, and 3 mM.

### 2. Rheological tests

The storage and loss moduli (G' and G", respectively) of h9e hydrogels were determined on a C-VOR 150 rheometer system (Malvern instruments, Malvern, Worcestershire WR141XZ, United Kingdom) with a 20-mm diameter parallel plate geometry and 500 µm gap size at 37 °C. The methods for hydrogel formation test and shear-thinning and self-healing test were the same as the methods of cancer cell medium mentioned above. After 24 hours of hydrogel formation, 2 mM peptide hydrogel was diluted 20 fold by adding cell culture medium and thorough mixing by pipette. The diluted solution was transferred to rheometer system and tested under 1 Hz frequency and 1% shear strain at 4 °C for 1 hour.

The stability of h9e-2i medium hydrogel was tested. A plastic tray was placed on the bottom of the well plate. Next, 1 wt % h9e solution was mixed with 2i medium at 1:1 (v:v) ratio in a centrifuge tube and then transferred into the well plate. There was 500 µl peptide hydrogel for each well. The well plate was then placed in an incubator for 1 hour. After hydrogel was stable, another 500 µl of 2i medium was slowly added on the top of hydrogel. The top medium was replaced every other day. For mechanical testing, the top 2i medium was removed and hydrogel was transferred to a dynamic rheometer using the tray. The stability of the peptide solution was also tested using 1wt% peptide solution stored in 4 °C refrigerator for up to 24 days. Part of the peptide solution was taken out and mixed with 2i medium with 1:1 (v:v) ratio on days 1, 6, 18 and 24. The hydrogel formation process was monitored under the single frequency (1 Hz) rheological test with 1 % shear strain for 1 hour.

### Results and Discussion

### 1. Peptide hydrogel formation in MSC medium

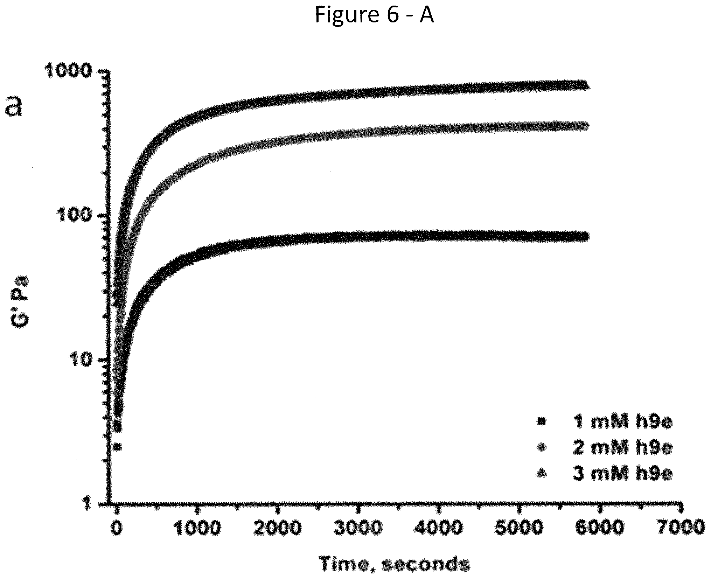

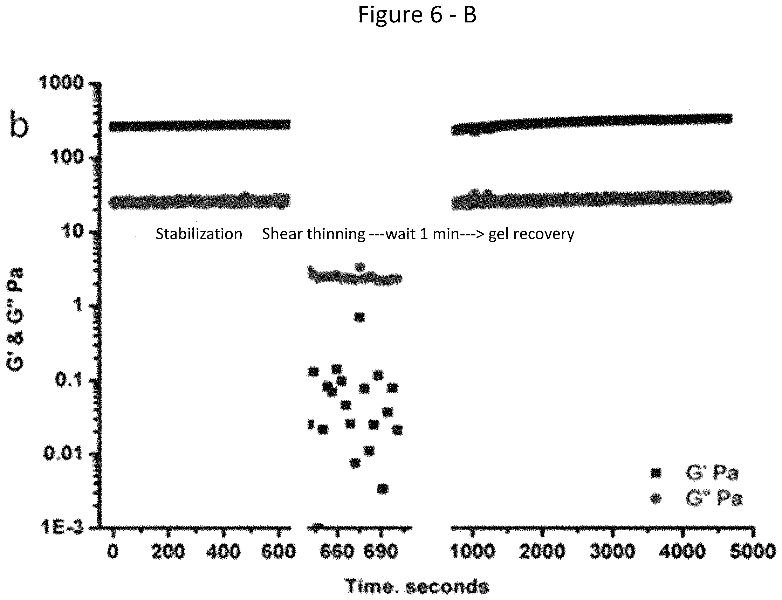

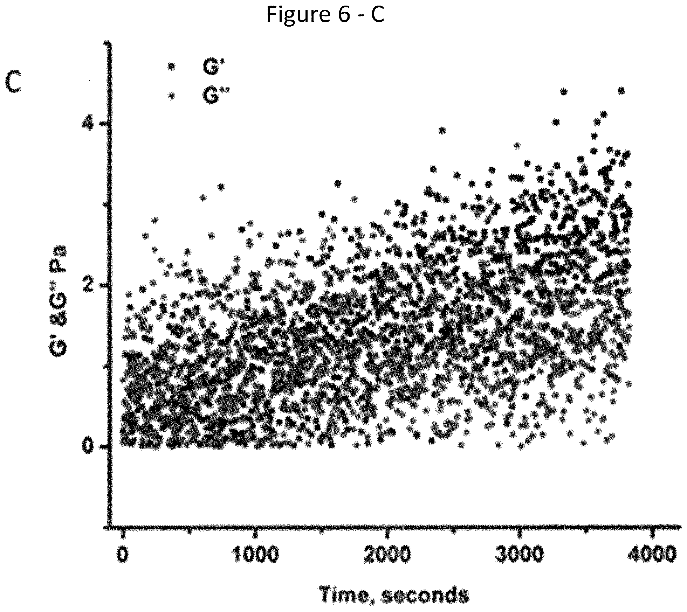

Three concentrations (1 mM, 2 mM and 3 mM) of h9e-MSC hydrogel were prepared by mixing 10 mM h9e solution with MSC cell culture medium at 1:9, 2:8 and 3:7 (v/v) ratios. The G' of the peptide-medium mixture was measured right after mixing. Figure 6a shows that G' of the hydrogel was increase with peptide concentration. The stable G' of 1 mM, 2 mM and 3 mM h9e-MSC hydrogel was 70 Pa, 350 Pa and 800 Pa respectively. The 2 mM h9e-MSC hydrogel was selected for shear-thinning and re-healing test (Figure 6b). A sample was stored at room temperature overnight and stabilized on the measuring system for 10 min. Under the 500% shear strain, the hydrogel was disturbed into liquid form with G' lower than 0.2 Pa. After the shear ceased for 1 min, greater than 90% of gel strength was recovered. In the following one hour 1% shear strain recovery test, 100% of the hydrogel mechanical strength was recovered within a few minutes (Figure 6b). To determine the cell recovery capability of this h9e-MSC hydrogel, cell medium was added to a stable hydrogel. After thorough mixing by pipette, the peptide concentration of the hydrogel was diluted 20-fold from 2 mM to 0.1 mM. The diluted solution was then transferred to a rheometer for single frequency testing. Figure 6c shows that during the 1 hour test, both the G' and G" of the solution was lower than 4 Pa, presenting as a very low viscous solution. This phenomenon ensures h9e- MCS hydrogel can be converted into liquid form and allows cell isolation after 3D cell culture.

### 2. Peptide hydrogel formation in 2i medium

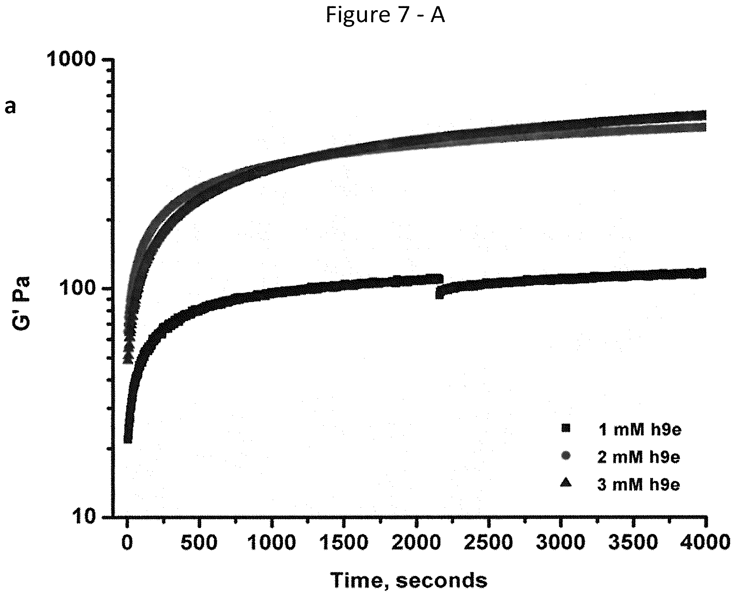

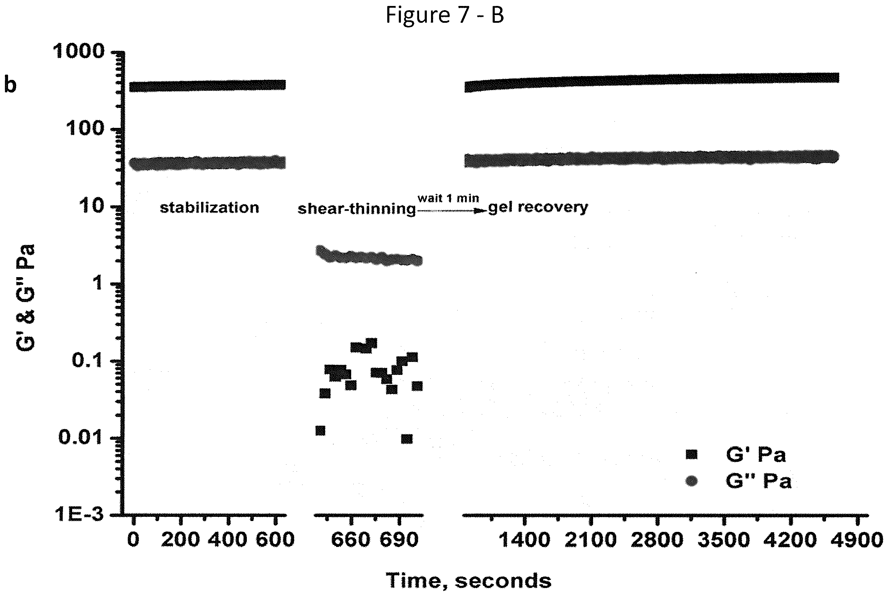

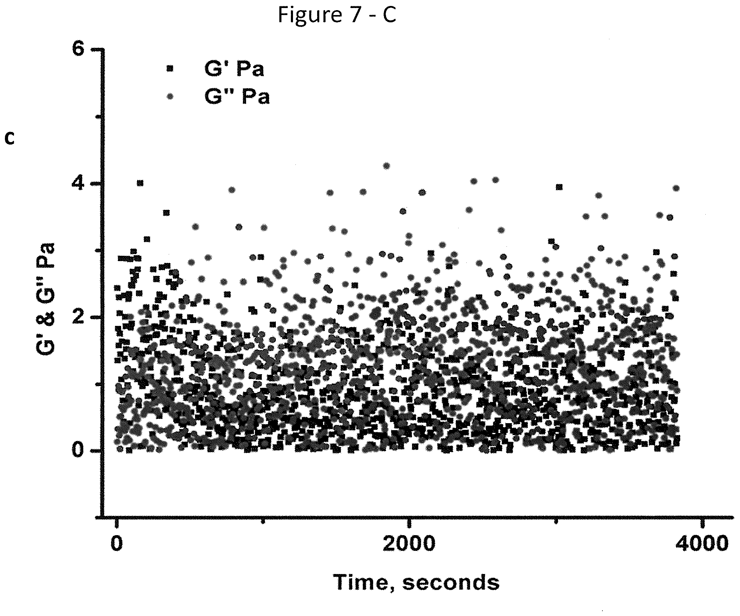

Three concentrations (1 mM, 2 mM and 3 mM) of h9e-2i hydrogel were prepared in the same way as h9e-MSC hydrogel. Figure 7a shows that G' of 1 mM is about 100 Pa. Hydrogels with 2 mM and 3 mM peptide concentration in 2i medium each have similar stable G' of 600 Pa. The 2 mM h9e-2i hydrogel was selected for shear-thinning and re-healing test (Figure 7b) by using the same method described for the h9e-MSC hydrogel. After the hydrogel was sheared into a liquid state (G' < 0.2 Pa), the G' of hydrogel was rapidly recovered after shear force was ceased. The mechanical strength of the hydrogel was 100% restored within a few minutes of the 1 hour 1% shear strain recovery test (Figure 7b). Furthermore, we also diluted the 2 mM h9e-2i hydrogel to a 0.1 mM concentration and demonstrated this method could be used to convert h9e-2i hydrogel into liquid form for cell recovery (Figure 7c).

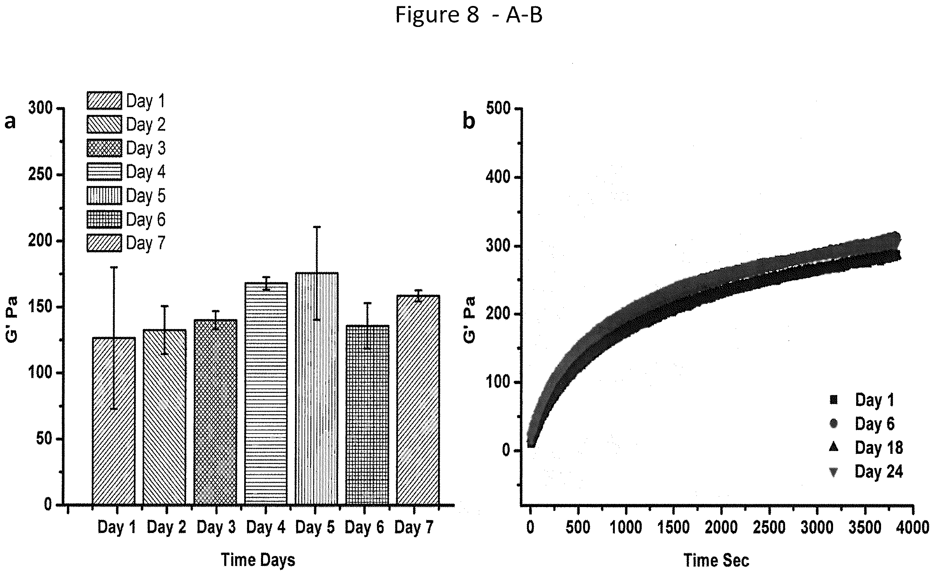

Moreover, the stability of h9e-2i hydrogel was also tested. Figure 8a shows the G' of h9e-2i hydrogel was stable around 130-160 Pa during 7 days. On the other hand, the stability of h9e solution was tested by monitoring the hydrogel formation of h9e-2i mixture after the h9e solution was stored at 4 °C for 1, 6, 18 and 24 days. Figure 8b shows that after 24 days storage, the h9e solution could still from a hydrogel when mixed with 2i medium and presented the same rate of hydrogel formation and stable G' value. A recent study indicated that the stability of h9e solution could exceed 2 months.

## Claims

1. A peptide-albumin hydrogel having an albumin-triggered self-assembling, 3-dimensional nanofiber matrix, said nanofiber matrix comprising a linear amphiphilic peptide and albumin, wherein said nanofiber matrix comprises a 3-dimensional nanofibrous network made up of said amphiphilic peptide and said albumin, wherein said peptide comprises a terminal hydrophobic region, a central turning region, and a terminal hydrophilic region.

2. The peptide-albumin hydrogel of claim 1, wherein said hydrogel is reversible.

3. The peptide-albumin hydrogel of claim 2, wherein said hydrogel has a % recovery of at least 60% in less than 10 minutes after destruction of said 3-dimensional nanofiber matrix by shear thinning.

4. The peptide-albumin hydrogel of claim 1, wherein said hydrogel has a storage modulus of from 50 Pa to 10,000 Pa at a pH of 7 and a temperature of from 20-25°C.

5. The peptide-albumin hydrogel of claim 1, wherein the weight ratio of peptide to albumin is from 100:1 to 1:100.

6. The peptide-albumin hydrogel of claim 1, wherein said hydrophilic region is derived from a β-spiral motif of spider flagelliform silk protein, and said hydrophobic and turning regions are derived from the third trans-membrane segment of subunit IV in the dihydropyridine sensitive human muscle L-type calcium channel.

7. The peptide-albumin hydrogel of claim 1, further comprising an active agent encapsulated in said 3-dimensional nanofiber matrix.

8. The peptide-albumin hydrogel of claim 1, further comprising cell medium and cells cultured in said 3-dimensional nanofiber matrix.

9. A method of forming a peptide-albumin hydrogel, said method comprising:
providing a peptide solution comprising a linear peptide dispersed, dissolved, or suspended in a solvent system, wherein said peptide is amphiphilic and comprises a terminal hydrophobic region, a central turning region, and a terminal hydrophilic region; and
mixing a source of albumin with said peptide solution at room temperature to form a peptide-albumin solution, wherein said peptide and albumin self-assemble into said peptide-albumin hydrogel without adjusting the pH, temperature, salt, or ion composition of said peptide-albumin solution, wherein said hydrogel has a nanofiber matrix comprising said peptide and said albumin, wherein said nanofiber matrix comprises a 3-dimensional nanofibrous network made up of said peptide and said albumin.

10. The method of claim 9, wherein the said peptide solution has a pH of from 6 to 8.

11. The method of claim 9, wherein said solvent system includes a solvent selected from the group consisting of sodium bicarbonate, sodium hydroxide, potassium hydroxide, and mixtures thereof in water.

12. The method of claim 9, wherein said hydrogel is formed in less than 120 minutes after said mixing.

13. The method of claim 9, wherein said albumin is selected from the group consisting of isolated, extracted, or purified albumin from plant or animal sources, synthesized albumin, derivatives thereof, and mixtures thereof.

14. The method of claim 9, wherein said source of albumin is selected from the group consisting of purified albumin, serum, serum-supplemented cell media, and combinations thereof.

15. The method of claim 9, wherein said source of albumin is mixed with a solvent system to form a solution comprising said albumin before mixing with said peptide solution.

16. The method of claim 9, wherein said peptide-albumin solution has a substantially neutral pH.

17. The method of claim 9, wherein said source of albumin is serum-supplemented cell media, said cell media comprising cells, wherein said cells are encapsulated by said peptide-albumin hydrogel, said method further comprising culturing said cells in said hydrogel.

18. The method of claim 17, further comprising applying said hydrogel to a cell culture plate or microwell for cell culturing.

19. The method of claim 18, further comprising covering said hydrogel with cell media and incubating said hydrogel containing said cells under cell culture conditions.

20. The method of claim 17, further comprising isolating said cultured cells from said hydrogel.

21. The method of claim 20, wherein said isolating comprises:
subjecting said hydrogel to a mechanical force to disrupt the hydrogel matrix;
diluting said hydrogel with additional cell media; and separating said cultured cells from said hydrogel.

22. The method of claim 21, wherein said mechanical force is selected from the group consisting of pipetting, centrifugation, vibration, injection, filtration, spraying, and combinations thereof.

23. The method of claim 9, further comprising mixing an active agent with said peptide solution and said source of albumin, said active agent being encapsulated in said peptide-albumin hydrogel after said self-assembly.

24. A peptide-albumin hydrogel formed according to the method of claim 9.

25. A peptide-albumin hydrogel according to any of claims 1-8 for use in a method of delivering an active agent to a patient, said method comprising administering to said patient the peptide-albumin hydrogel, wherein said active agent is encapsulated in said hydrogel matrix.

## Patentansprüche

1. Peptid-Albumin-Hydrogel mit einer durch Albumin induzierten, selbstorganisierenden, dreidimensionalen Nanofasermatrix, wobei die Nanofasermatrix, wobei die Nanofasermatrix ein lineares, amphiphiles Peptid und Albumin umfasst, und wobei die Nanofasermatrix ein dreidimensionales Nanofasernetzwerk umfasst, das aus dem amphiphilen Peptid und dem Albumin zusammengesetzt ist, wobei das Peptid einen endständigen hydrophoben Bereich, einen zentralen Drehbereich und einen endständigen hydrophilen Bereich umfasst.

2. Peptid-Albumin-Hydrogel nach Anspruch 1, wobei das Hydrogel reversibel ist.

3. Peptid-Albumin-Hydrogel nach Anspruch 2, wobei das Hydrogel eine prozentuale Rückgewinnung von mindestens 60 % in weniger als 10 Minuten nach Zerstörung der dreidimensionalen Nanofasermatrix durch Scherverdünnung aufweist.

4. Peptid-Albumin-Hydrogel nach Anspruch 1, wobei das Hydrogel einen Speichermodul von 50 Pa bis 10.000 Pa bei einem pH-Wert von 7 und einer Temperatur von 20-25°C aufweist.

5. Peptid-Albumin-Hydrogel nach Anspruch 1, wobei das Gewichtsverhältnis von Peptid zu Albumin von 100:1 bis 1:100 beträgt.

6. Peptid-Albumin-Hydrogel nach Anspruch 1, wobei die hydrophile Region von einem β-Spiralmotiv aus Spinnen-Flagelliform-Seidenprotein abgeleitet ist, und wobei die hydrophoben und drehenden Regionen vom dritten Transmembransegment der Untereinheit IV des Dihydropyridin-empfindlichen Kalziumkanal vom L-Typ des menschlichen Muskels abgeleitet sind.

7. Peptid-Albumin-Hydrogel nach Anspruch 1, das ferner einen Wirkstoff umfasst, der in die dreidimensionale Nanofasermatrix eingekapselt ist.

8. Peptid-Albumin-Hydrogel nach Anspruch 1, das ferner Zellmedium und Zellen umfasst, die in der dreidimensionalen Nanofasermatrix kultiviert werden.

9. Verfahren zur Herstellung eines Peptid-Albumin-Hydrogels, bei dem man :
eine Peptidlösung bereitstellt, die ein lineares Peptid umfasst, das in einem Lösungsmittelsystem dispergiert, gelöst oder suspendiert ist, wobei das Peptid amphiphil ist und einen endständigen hydrophoben Bereich, einen zentralen Drehbereich und einen endständigen hydrophilen Bereich umfasst; und
eine Albuminquelle mit der Peptidlösung bei Raumtemperatur mischt, um eine Peptid-Albumin-Lösung zu bilden, wobei sich das Peptid und das Albumin ohne Einstellen von pH-Wert, Temperatur, Salz oder Ionenzusammensetzung der Peptid-Albumin-Lösung selbst zu dem Peptid-Albumin-Hydrogel zusammensetzen, wobei das Hydrogel eine Nanofasermatrix aufweist, die das Peptid und das Albumin umfasst, wobei die Nanofasermatrix ein dreidimensionales Nanofasernetzwerk aufweist, das das aus Peptid und dem Albumin zusammengesetzt ist.

10. Verfahren nach Anspruch 9, wobei die Peptidlösung einen pH-Wert von 6 bis 8 aufweist.

11. Verfahren nach Anspruch 9, wobei das Lösungsmittelsystem ein Lösungsmittel enthält, das ausgewählt ist aus der Gruppe bestehend aus Natriumbicarbonat, Natriumhydroxid, Kaliumhydroxid und Mischungen davon in Wasser.

12. Verfahren nach Anspruch 9, wobei das Hydrogel in weniger als 120 Minuten nach dem Mischen gebildet wird.

13. Verfahren nach Anspruch 9, wobei das Albumin ausgewählt ist aus der Gruppe bestehend aus isoliertem, extrahiertem oder gereinigtem Albumin aus pflanzlichen oder tierischen Quellen, synthetisiertem Albumin, Derivaten davon und Mischungen davon.

14. Verfahren nach Anspruch 9, wobei die Albuminquelle ausgewählt ist aus der Gruppe bestehend aus gereinigtem Albumin, Serum, Serum-supplementierten Zellmedien und Kombinationen davon.

15. Verfahren nach Anspruch 9, wobei die Albuminquelle vor Mischen mit der Peptidlösung mit einem Lösungsmittelsystem vermischt wird, um eine Lösung zu bilden, die das Albumin umfasst.

16. Verfahren nach Anspruch 9, wobei die Peptid-Albumin-Lösung einen im Wesentlichen neutralen pH-Wert aufweist.

17. Verfahren nach Anspruch 9, wobei die Albuminquelle Serumsupplementierte Zellmedien sind, wobei die Zellmedien Zellen umfassen, wobei die Zellen durch das Peptid-Albumin-Hydrogel eingekapselt sind, und wobei das Verfahren ferner das Kultivieren der Zellen in dem Hydrogel umfasst.

18. Verfahren nach Anspruch 17, das ferner das Aufbringen des Hydrogels auf eine Zellkulturplatte oder Mikrotiterplatte zur Zellkultivierung umfasst.

19. Verfahren nach Anspruch 18, das ferner das Bedecken des Hydrogels mit Zellmedien und das Inkubieren des die Zellen enthaltenden Hydrogels unter Zellkulturbedingungen umfasst.

20. Verfahren nach Anspruch 17, das ferner die Isolierung der kultivierten Zellen aus dem Hydrogel umfasst.

21. Verfahren nach Anspruch 20, bei dem man für die Isolierung:
das Hydrogel einer mechanischen Kraft unterzieht, um die Hydrogelmatrix zu zerstören;
das Hydrogel mit zusätzlichen Zellmedien verdünnt; und die kultivierten Zellen von dem Hydrogel abtrennt.

22. Verfahren nach Anspruch 21, wobei die mechanische Kraft ausgewählt ist aus der Gruppe bestehend aus Pipettieren, Zentrifugieren, Vibration, Injektion, Filtration, Sprühen und Kombinationen davon.

23. Verfahren nach Anspruch 9, bei dem man ferner einen Wirkstoff mit der Peptidlösung und der Albuminquelle mischt, wobei der Wirkstoff nach der Selbstzusammensetzung in das Peptid-Albumin-Hydrogel eingekapselt ist.

24. Peptid-Albumin-Hydrogel, das nach dem Verfahren von Anspruch 9 gebildet ist.

25. Peptid-Albumin-Hydrogel nach einem der Ansprüche 1-8 zur Verwendung in einem Verfahren zur Abgabe eines Wirkstoffs an einen Patienten, wobei das Verfahren die Verabreichung des Peptid-Albumin-Hydrogels an den Patienten umfasst, wobei der Wirkstoff in der Hydrogelmatrix eingekapselt ist.

## Revendications

1. Hydrogel de peptide-albumine comportant une matrice tridimensionnelle de nanofibres, à auto-assemblage déclenché par l'albumine, ladite matrice de nanofibres comprenant un peptide amphiphile linéaire et de l'albumine, dans lequel ladite matrice de nanofibres comprend un réseau tridimensionnel nanofibreux constitué dudit peptide amphiphile et de ladite albumine, et dans lequel ledit peptide comprend une région hydrophobe terminale, une région centrale pivotante, et une région hydrophile terminale.

2. Hydrogel de peptide-albumine selon la revendication 1, dans lequel ledit hydrogel est réversible.

3. Hydrogel de peptide-albumine selon la revendication 2, dans lequel ledit hydrogel a un pourcentage de récupération d'au moins 60 % en moins de 10 minutes après destruction de ladite matrice tridimensionnelle de nanofibres par diminution de la viscosité sous effet de cisaillement.

4. Hydrogel de peptide-albumine selon la revendication 1, dans lequel ledit hydrogel a un module de stockage de 50 Pa à 10 000 Pa à un pH de 7 et à une température de 20 - 25 °C.

5. Hydrogel de peptide-albumine selon la revendication 1, dans lequel le rapport pondéral du peptide à l'albumine vaut de 100 : 1 à 1 : 100.

6. Hydrogel de peptide-albumine selon la revendication 1, dans lequel ladite région hydrophile est issue d'un motif β-spiral de protéine de soie flagelliforme d'araignée, et lesdites régions hydrophobe et pivotante sont issues du troisième segment transmembranaire de la sous-unité IV dans le canal calcique de type L de muscle humain sensible à la dihydropyridine.

7. Hydrogel de peptide-albumine selon la revendication 1, comprenant en outre un agent actif encapsulé dans ladite matrice tridimensionnelle de nanofibres.

8. Hydrogel de peptide-albumine selon la revendication 1, comprenant en outre un milieu de culture de cellules et des cellules cultivées dans ladite matrice tridimensionnelle de nanofibres.

9. Procédé de formation d'un hydrogel de peptide-albumine, ledit procédé comprenant les étapes consistant à :
fournir une solution de peptide comprenant un peptide linéaire dispersé, dissous ou mis en suspension dans un système de solvant, ledit peptide étant amphiphile et comprenant une région hydrophobe terminale, une région centrale pivotante, et une région hydrophile terminale ; et
mélanger une source d'albumine avec ladite solution de peptide, à la température ambiante, pour former une solution de peptide-albumine, ledit peptide et ladite albumine s'auto-assemblant en ledit hydrogel de peptide-albumine sans ajustement du pH, de la température, de la composition en sels ou en ions de ladite solution de peptide-albumine, ledit hydrogel comportant une matrice de nanofibres comprenant ledit peptide et ladite albumine, ladite matrice de nanofibres comprenant un réseau tridimensionnel nanofibreux constituée dudit peptide et de ladite albumine.

10. Procédé selon la revendication 9, dans lequel ladite solution de peptide a un pH de 6 à 8.

11. Procédé selon la revendication 9, dans lequel ledit système de solvant inclut un solvant choisi dans l'ensemble constitué par le bicarbonate de sodium, l'hydroxyde de sodium, l'hydroxyde de potassium et des mélanges de ceux-ci dans de l'eau.

12. Procédé selon la revendication 9, dans lequel ledit hydrogel est formé en moins de 120 minutes après ledit mélange.

13. Procédé selon la revendication 9, dans lequel ladite albumine est choisie dans l'ensemble constitué par une albumine isolée, extraite ou purifiée provenant de sources animales ou végétales, une albumine de synthèse, des dérivés d'une telle albumine et des mélanges de telles albumines et de tels dérivés.

14. Procédé selon la revendication 9, dans lequel ladite source d'albumine est choisie dans l'ensemble constitué par l'albumine purifiée, le sérum, des milieux de culture de cellules additionnés de sérum et des associations de telles sources.

15. Procédé selon la revendication 9, dans lequel on mélange ladite source d'albumine avec un système de solvant pour former une solution comprenant ladite albumine, avant de la mélanger avec ladite solution de peptide.

16. Procédé selon la revendication 9, dans lequel ladite solution de peptide-albumine a un pH essentiellement neutre.

17. Procédé selon la revendication 9, dans lequel ladite source d'albumine consiste en des milieux de culture de cellules additionnés de sérum, lesdits milieux de culture de cellules comprenant des cellules, lesdites cellules étant encapsulées par ledit hydrogel de peptide-albumine, ledit procédé comprenant en outre la culture desdites cellules dans ledit hydrogel.

18. Procédé selon la revendication 17, comprenant en outre l'application dudit hydrogel sur une plaque de culture de cellules ou un micropuits pour la culture de cellules.

19. Procédé selon la revendication 18, comprenant en outre les étapes consistant à recouvrir ledit hydrogel avec un milieu de culture et à mettre à incuber ledit hydrogel contenant lesdites cellules, dans des conditions de culture de cellules.

20. Procédé selon la revendication 17, comprenant en outre l'isolement desdites cellules cultivées à partir dudit hydrogel.

21. Procédé selon la revendication 20, dans lequel ledit isolement comprend les étapes consistant à :
soumettre ledit hydrogel à une force mécanique pour rompre la matrice d'hydrogel ;
diluer ledit hydrogel avec du milieu de culture de cellules supplémentaire ; et séparer lesdites cellules cultivées dudit hydrogel.

22. Procédé selon la revendication 21, dans lequel ladite force mécanique est choisie dans l'ensemble constitué par le pipetage, la centrifugation, la vibration, l'injection, la filtration, la pulvérisation et des associations de telles forces.

23. Procédé selon la revendication 9, comprenant en outre le mélange d'un agent actif avec ladite solution de peptide et ladite source d'albumine, ledit agent actif étant encapsulé dans ledit hydrogel de peptide-albumine après ledit auto-assemblage.

24. Hydrogel de peptide-albumine formé conformément au procédé selon la revendication 9.

25. Hydrogel de peptide-albumine selon l'une quelconque des revendications 1 à 8, pour utilisation dans un procédé d'apport d'un agent actif à un patient, ledit procédé comprenant l'administration audit patient de l'hydrogel de peptide-albumine, ledit agent actif étant encapsulé dans ladite matrice d'hydrogel.


### SEQUENCE LISTING

<110> Kansas State University Research Foundation Huang, Hongzhou Sun, Xiuzhi Susan
<120> PEPTIDE-ALBUMIN HYDROGEL PROPERTIES AND ITS APPLICATIONS
<130> 44873-PCT
<140> EP13861358.3
<150> US 61/734,708
   <151> 2012-12-07
<160> 54
<170> PatentIn version 3.5
<210> 1
   <211> 19
   <212> PRT
   <213> Artificial Sequence
<220>
   <223> Derivative of spider flagelliform silk protein and a trans-membrane segment of subunit IV in the dihydropyridine sensitive human muscle L-type calcium channel
<400> 1
   Phe Leu Ile Val Ile Gly Ser Ile Ile Gly Pro Gly Gly Asp Gly Pro
   1               5                   10                  15
   Gly Gly Asp
<210> 2
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 2
   Phe Leu Ile Val Ile
   1               5
<210> 3
   <211> 4
   <212> PRT
   <213> Artificial Sequence
<220>
   <223> Peptide turning region derived from the third trans-membrane segment of subunit IV in the dihydropyridine sensitive human muscle L-type calcium channel
<400> 3
   Gly Ser Ile Ile
   1
<210> 4
   <211> 10
   <212> PRT
   <213> Artificial Sequence
<220>
   <223> Derivative of spider flagelliform silk protein
<400> 4
   Gly Pro Gly Gly Asp Gly Pro Gly Gly Asp
   1               5                   10
<210> 5
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 5
   Gly Leu Ile Val Ile
   1               5
<210> 6
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 6
   Pro Leu Ile Val Ile
   1               5
<210> 7
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 7
   Asp Leu Ile Val Ile
   1 5
<210> 8
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 8
   Val Leu Ile Val Ile
   1 5
<210> 9
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 9
   Ile Leu Ile Val Ile
   1 5
<210> 10
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 10
   Leu Leu Ile Val Ile
   1 5
<210> 11
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 11
   Ala Leu Ile Val Ile
   1 5
<210> 12
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 12
   Phe Gly Ile Val Ile
   1 5
<210> 13
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 13
   Phe Pro Ile Val Ile
   1 5
<210> 14
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 14
   Phe Asp Ile Val Ile
   1 5
<210> 15
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 15
   Phe Val Ile Val Ile
   1 5
<210> 16
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 16
   Phe Ile Ile Val Ile
   1 5
<210> 17
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 17
   Phe Ala Ile Val Ile
   1 5
<210> 18
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 18
   Phe Leu Gly Val Ile
   1 5
<210> 19
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 19
   Phe Leu Pro Val Ile
   1 5
<210> 20
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 20
   Phe Leu Asp Val Ile
   1               5
<210> 21
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 21
   Phe Leu Val Ile Val
   1               5
<210> 22
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 22
   Phe Leu Ala Val Ile
   1               5
<210> 23
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 23
   Phe Leu Ile Gly Ile
   1               5
<210> 24
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 24
   Phe Leu Ile Pro Ile
   1               5
<210> 25
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 25
   Phe Leu Ile Asp Ile
   1               5
<210> 26
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 26
   Phe Leu Ile Ile Ile
   1               5
<210> 27
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 27
   Phe Leu Ile Leu Ile
   1               5
<210> 28
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 28
   Phe Leu Ile Ala Ile
   1               5
<210> 29
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 29
   Phe Leu Ile Val Gly
   1               5
<210> 30
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 30
   Phe Leu Ile Val Pro
   1               5
<210> 31
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 31
   Phe Leu Ile Val Asp
   1               5
<210> 32
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 32
   Phe Leu Ile Val Val
   1 5
<210> 33
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 33
   Phe Leu Ile Val Leu
   1 5
<210> 34
   <211> 5
   <212> PRT
   <213> Artificial Sequence
<220>
   <223> Hydrophobic region derived from the third trans-membrane segment of subunit IV in the dihydropyridine sensitive human muscle L-type calcium channel
<400> 34
   Phe Leu Ile Val Ala
   1 5
<210> 35
   <211> 5
   <212> PRT
   <213> Artificial Sequence
<220>
   <223> Derivative of spider flagelliform silk protein
<220>
   <221> MISC_FEATURE
   <222> (3)..(4)
   <223> each Xaa is individually G, A, D, R, Q, E, S, T, K, or P
<400> 35
   Gly Pro Xaa Xaa Asp
   1 5
<210> 36
   <211> 5
   <212> PRT
   <213> Artificial Sequence
<220>
   <223> Derivative of spider flagelliform silk protein
<220>
   <221> MISC_FEATURE
   <222> (2)..(3)
   <223> each Xaa is individually G, A, D, R, Q, E, S, T, K, or P
<400> 36
   Gly Xaa Xaa Pro Asp
   1               5
<210> 37
   <211> 5
   <212> PRT
   <213> Artificial Sequence
<220>
   <223> Derivative of spider flagelliform silk protein
<220>
   <221> MISC_FEATURE
   <222> (2)..(2)
   <223> each Xaa is individually G, A, D, R, Q, E, S, T, K or P
<220>
   <221> MISC_FEATURE
   <222> (4)..(4)
   <223> each Xaa is individually G, A, D, R, Q, E, S, T, K or P
<400> 37
   Gly Xaa Pro Xaa Asp
   1               5
<210> 38
   <211> 10
   <212> PRT
   <213> Artificial Sequence
<220>
   <223> Derivative of spider flagelliform silk protein
<220>
   <221> MISC_FEATURE
   <222> (4)..(4)
   <223> Xaa is G or A
<220>
   <221> MISC_FEATURE
   <222> (9)..(9)
   <223> Xaa is G or A
<400> 38
   Gly Pro Gly Xaa Asp Gly Pro Gly Xaa Asp
   1               5                   10
<210> 39
   <211> 10
   <212> PRT
   <213> Artificial Sequence
<220>
   <223> Derivative of spider flagelliform silk protein
<220>
   <221> MISC_FEATURE
   <222> (4)..(4)
   <223> Xaa is A, G, V, I, or L
<220>
   <221> MISC_FEATURE
   <222> (7)..(7)
   <223> Xaa is P, A, G, V, I, or L
<220>
   <221> MISC_FEATURE
   <222> (8)..(9)
   <223> each Xaa is individually A, G, V, I, or L
<400> 39
   Gly Pro Gly Xaa Asp Gly Xaa Xaa Xaa Asp
   1               5                   10
<210> 40
   <211> 5
   <212> PRT
   <213> Artificial Sequence
<220>
   <223> Derivative of spider flagelliform silk protein
<220>
   <221> MISC_FEATURE
   <222> (4)..(4)
   <223> Xaa is A, G, V, I, or L
<400> 40
   Gly Pro Gly Xaa Asp
   1               5
<210> 41
   <211> 4
   <212> PRT
   <213> Artificial Sequence
<220>
   <223> Peptide turning region
<400> 41
   Gly Gly Gly Gly
   1
<210> 42
   <211> 4
   <212> PRT
   <213> Artificial Sequence
<220>
   <223> Peptide turning region
<220>
   <221> VARIANT
   <222> (2)..(2)
   <223> /replace= "Thr"
<220>
   <221> MISC_FEATURE
   <222> (3)..(4)
   <223> each Xaa is individually G, I, V, A, L, or S
<400> 42
   Gly Ser Xaa Xaa
   1
<210> 43
   <211> 4
   <212> PRT
   <213> Artificial Sequence
<220>
   <223> Peptide turning region
<220>
   <221> MISC_FEATURE
   <222> (1)..(1)
   <223> each Xaa is individually G, I, V, A, L, or S
<220>
   <221> VARIANT
   <222> (3)..(3)
   <223> /replace= "Thr"
<220>
   <221> MISC_FEATURE
   <222> (4)..(4)
   <223> each Xaa is individually G, I, V, A, L, or S
<400> 43
   Xaa Gly Ser Xaa
   1
<210> 44
   <211> 4
   <212> PRT
   <213> Artificial Sequence
<220>
   <223> Peptide turning region
<220>
   <221> MISC_FEATURE
   <222> (1)..(2)
   <223> each Xaa is individually G, I, V, A, L, or S
<220>
   <221> VARIANT
   <222> (4)..(4)
   <223> /replace= "Thr"
<400> 44
   Xaa Xaa Gly Ser
   1
<210> 45
   <211> 4
   <212> PRT
   <213> Artificial Sequence
<220>
   <223> Peptide turning region
<220>
   <221> VARIANT
   <222> (1)..(1)
   <223> /replace= "Thr"
<220>
   <221> MISC_FEATURE
   <222> (3)..(4)
   <223> each Xaa is individually G, I, V, A, L, or S
<400> 45
   Ser Gly Xaa Xaa
   1
<210> 46
   <211> 4
   <212> PRT
   <213> Artificial Sequence
<220>
   <223> Peptide turning region
<220>
   <221> MISC_FEATURE
   <222> (1)..(1)
   <223> each Xaa is individually G, I, V, A, L, or S
<220>
   <221> VARIANT
   <222> (2)..(2)
   <223> /replace= "Thr"
<220>
   <221> MISC_FEATURE
   <222> (4)..(4)
   <223> each Xaa is individually G, I, V, A, L, or S
<400> 46
   Xaa Ser Gly Xaa
   1
<210> 47
   <211> 4
   <212> PRT
   <213> Artificial Sequence
<220>
   <223> Peptide turning region
<220>
   <221> MISC_FEATURE
   <222> (1)..(2)
   <223> each Xaa is individually G, I, V, A, L, or S
<220>
   <221> VARIANT
   <222> (3)..(3)
   <223> /replace= "Thr"
<400> 47
   Xaa Xaa Ser Gly
   1
<210> 48
   <211> 4
   <212> PRT
   <213> Artificial Sequence
<220>
   <223> Peptide turning region
<220>
   <221> MISC_FEATURE
   <222> (2)..(2)
   <223> each Xaa is individually G, I, V, A, L, or S
<220>
   <221> VARIANT
   <222> (3)..(3)
   <223> /replace= "Thr"
<220>
   <221> MISC_FEATURE
   <222> (4)..(4)
   <223> each Xaa is individually G, I, V, A, L, or S
<400> 48
   Gly Xaa Ser Xaa
   1
<210> 49
   <211> 4
   <212> PRT
   <213> Artificial Sequence
<220>
   <223> Peptide turning region
<220>
   <221> MISC_FEATURE
   <222> (1)..(1)
   <223> each Xaa is individually G, I, V, A, L, or S
<220>
   <221> MISC_FEATURE
   <222> (3)..(3)
   <223> each Xaa is individually G, I, V, A, L, or S
<220>
   <221> VARIANT
   <222> (4)..(4)
   <223> /replace= "Thr"
<400> 49
   Xaa Gly Xaa Ser
   1
<210> 50
   <211> 4
   <212> PRT
   <213> Artificial Sequence
<220>
   <223> Peptide turning region
<220>
   <221> VARIANT
   <222> (1)..(1)
   <223> /replace= "Thr"
<220>
   <221> MISC_FEATURE
   <222> (2)..(2)
   <223> each Xaa is individually G, I, V, A, L, or S
<220>
   <221> MISC_FEATURE
   <222> (4)..(4)
   <223> each Xaa is individually G, I, V, A, L, or S
<400> 50
   Ser Xaa Gly Xaa
   1
<210> 51
   <211> 4
   <212> PRT
   <213> Artificial Sequence
<220>
   <223> Peptide turning region
<220>
   <221> MISC_FEATURE
   <222> (1)..(1)
   <223> each Xaa is individually G, I, V, A, L, or S
<220>
   <221> VARIANT
   <222> (2)..(2)
   <223> /replace= "Thr"
<220>
   <221> MISC_FEATURE
   <222> (3)..(3)
   <223> each Xaa is individually G, I, V, A, L, or S
<400> 51
   Xaa Ser Xaa Gly
   1
<210> 52
   <211> 4
   <212> PRT
   <213> Artificial Sequence
<220>
   <223> Peptide turning region
<220>
   <221> MISC_FEATURE
   <222> (2)..(3)
   <223> each Xaa is individually G, I, V, A, L, or S
<220>
   <221> VARIANT
   <222> (4)..(4)
   <223> /replace= "Thr"
<400> 52
   Gly Xaa Xaa Ser
   1
<210> 53
   <211> 4
   <212> PRT
   <213> Artificial Sequence
<220>
   <223> Peptide turning region
<220>
   <221> VARIANT
   <222> (1)..(1)
   <223> /replace= "Thr"
<220>
   <221> MISC_FEATURE
   <222> (2)..(3)
   <223> each Xaa is individually G, I, V, A, L, or S
<400> 53
   Ser Xaa Xaa Gly
   1
<210> 54
   <211> 4
   <212> PRT
   <213> Artificial Sequence
<220>
   <223> Peptide turning region derived from the third trans-membrane segment of subunit IV in the dihydropyridine sensitive human muscle L-type calcium channel
<220>
   <221> MISC_FEATURE
   <222> (1)..(1)
   <223> Xaa is G, I, or V
<220>
   <221> VARIANT
   <222> (2)..(2)
   <223> /replace= "Thr"
<220>
   <221> MISC_FEATURE
   <222> (3)..(4)
   <223> each Xaa is individually G, I, V, A, or L
<400> 54
   Xaa Ser Xaa Xaa
   1